# EUROPEAN PATENT APPLICATION

(11) **EP 4 130 285 A1**
(43) Date of publication of application: **08.02.2023**
(21) Application number: 21774853.2
(22) Date of filing: 25.03.2021
(51) Int. Cl.: C12P 7/62, C12R 1/01, C12N 1/20

(54) **METHOD FOR PRODUCING PHA USING SEA WATER**

(30) Priority: 27.03.2020 JP 2020058908
(71) Applicant: Sumitomo Forestry Co., Ltd., Tokyo 100-8270 (JP)
(72) Inventor: INO Kotaro, Tokyo 100-8270 (JP); KADOTA Kenichi, Tokyo 100-8270 (JP)
(74) Representative: Henkel & Partner mbB
(86) International application number: PCT/JP2021/012646
(87) International publication number: WO 2021/193847

(57) **Abstract**

A method for producing a polyhydroxyalkanoate that includes the following steps: (a) a step for culturing halobacteria with a culture medium containing sea water; and (b) a step for obtaining a polyhydroxyalkanoate as product of the culture.

## Description

### Technical Field

The present invention relates to a novel production method for a PHA (polyhydroxyalkanoic acid) copolymer. In addition, the present invention also relates to a method for using surplus seawater.

### Background Art

In the latter half of the 20th century, the technology for producing plastics from petroleum developed rapidly, and the spread of the plastic drastically changed people's lives. Numerous Nobel Prizes for plastics (nylon by Carothers, polyethylene, polypropylene by Ziegler-Natta, etc.) also have been awarded.

In recent years, 10.75 million tons of plastics have been produced annually in Japan alone (2016). On the other hand, most plastics are made from petroleum, causing problems such as the impact of carbon dioxide emissions at the time of disposal on global warming and the stable supply of petroleum resources. Furthermore, since ordinary plastics are not decomposed in nature once they are released into the environment, the problem of microplastics has been exacerbated by the fact that the plastics float in the sea as they are.

The solutions to such problems include biomass plastics and biodegradable plastics. The biomass plastic is a plastic made from raw materials that are not fossil fuel but biomass such as squeezed wastes of corns, sugarcanes, and the like, and wood, and the biodegradable plastic is a plastic which is decomposed into water and carbon dioxide by the power of microorganisms in the environment after use.

Polyhydroxy alkanoic acids (PHAs) are plastics stored, when certain microorganisms are fed with biomass as carbon sources (nutrients) such as sugars and vegetable oils, as energy storage substances; they are bioplastics that biodegrade in the environment after use and mitigate the environmental load.

Poly(3-hydroxybutanoic acid) (may be described as "P(3HB)") was the first discovered PHA. Non-Patent Document 1 discloses 3-hydroxyvaleric acid (may be referred to as "3HV") as a second component in addition to 3-hydroxybutanoic acid as a monomer component, and the like.

Techniques using seawater for synthesis of a PHA have also been known (Patent Document 2 and Patent Document 4).

### Prior Art Documents

### Patent Document

Patent Document 1: JP 2016-185087 A
Patent Document 2: JP 2018-133997 A
Patent Document 3: JP 2014-512194 A
Patent Document 4: JP 2012-210165 A

### Non Patent Document

Non-Patent Document 1: Macromolecular Bioscience 2007, 7, 218

### Summary of Invention

### Technical Problem

There is a constant demand for the method for producing a PHA. An object of the present invention is to provide a novel production method for the PHA.

### Solution to Problem

The present inventors have found, as a result of diligent efforts to solve the above problems, that it may be possible to produce a PHA by using a material that has never been used before, and, with further experimentation, have completed the present invention.

More specifically, the present invention relates to at least the following inventions:
[1] A production method for a polyhydroxyalkanoic acid, comprising:
   (a) culturing a microorganism by using a medium including seawater; and
   (b) obtaining the polyhydroxyalkanoic acid as a culture product in the culture.
[2] The production method according to [1], comprising NaCl other than NaCl derived from seawater in the medium.
[3] The production method according to [1] or [2], wherein a halophilic bacterium is a highly halophilic bacterium.
[4] The production method according to any one of [1] to [3], wherein the highly halophilic bacterium is Haloferax mediterranei.
[5] The production method according to any of [1] to [4], wherein the polyhydroxyalkanoic acid is a copolymer formed by random copolymerization of 3-hydroxybutanoic acid and 3-hydroxyvaleric acid.
[6] The production method according to [5], wherein a molar percentage of 3-hydroxyvaleric acid (3HV fraction) to the total moles of 3-hydroxybutanoic acid and 3-hydroxyvaleric acid constituting the copolymer is 5.0% to 40.0%.
[7] The production method according to [6], wherein the polyhydroxyalkanoic acid is obtained with a sugar as a carbon source selected from:
   (a) glucose and/or a derivative thereof alone,
   (b) glucose and a derivative thereof as well as one or more of xylose, cellobiose, glucuronic acid, arabinose, mannose, galactose, sucrose and derivatives thereof, or
   (c) one or more of xylose, cellobiose, glucuronic acid, arabinose, mannose, galactose, sucrose and a derivative thereof.
[8] The production method according to any one of [1] to [7], wherein the polyhydroxyalkanoic acid has a weight-average molecular weight of more than 3.61 million.
[9] The production method according to any one of [1] to [8], wherein the polyhydroxyalkanoic acid obtained has a weight-average molecular weight of 6 million or less.
[10] The production method according to any one of [1] to [9], wherein the seawater comprises concentrated seawater.
[11] The production method according to [10], wherein a salt concentration of the concentrated seawater is 3.5% to 20%.
[12] The production method according to any one of [1] to [11], wherein the medium comprises at least one of the following component or seawater:
   (i) artificial seawater elements,
   (ii) deep seawater, and
   (iii) natural seawater.
[13] Use of seawater in a production method for a polyhydroxyalkanoic acid, said production method comprising:
   (a) culturing a halophilic bacterium by using a medium comprising concentrated or unconcentrated seawater; and
   (b) obtaining the polyhydroxyalkanoic acid as a culture product in the medium.
[14] A production method for a polyhydroxyalkanoic acid comprising culturing a halophilic bacterium by using a medium comprising seawater to obtain the polyhydroxyalkanoic acid as a culture product,
   wherein a carbon source in the culture comprises a sugar and a furfural compound, the sugar being selected from:
   (a) glucose and/or a derivative thereof alone,
   (b) glucose and a derivative thereof as well as one or more of xylose, cellobiose, glucuronic acid, arabinose, mannose, galactose, sucrose, and derivatives thereof, or
   (c) one or more of xylose, cellobiose, glucuronic acid, arabinose, mannose, galactose, sucrose, and a derivative thereof,
   to obtain the polyhydroxy alkanoic acid.
[15] A method for culturing a halophilic bacterium by using a medium comprising seawater to control a molecular weight of the polyhydroxyalkanoic acid obtained as a culture product,
   wherein a carbon source in the culture comprises a sugar and a furfural compound, the sugar being selected from:
   (a) glucose and/or a derivative thereof alone,
   (b) glucose and a derivative thereof as well as one or more of xylose, cellobiose, glucuronic acid, arabinose, mannose, galactose, sucrose, and derivatives thereof, or
   (c) one or more of xylose, cellobiose, glucuronic acid, arabinose, mannose, galactose, sucrose, and a derivative thereof,
   thereby controlling a weight-average molecular weight of the polyhydroxyalkanoic acid produced.
[16] The production method according to any one of the above [1] to [15], further comprising NaCl that is not derived from artificial seawater elements, NaCl that is not derived from deep seawater, or NaCl that is not derived from natural seawater.
[17] The production method according to any one of the above [1] to [16], wherein the seawater is artificial seawater, and artificial seawater elements used in the artificial seawater is comprised in an amount greater than that normally used for preparing artificial seawater.

### Advantageous Effects of Invention

According to the present invention, a method for producing a PHA by using a material that has never been used before as a novel production method for the PHA, is provided.

There has been a report on the production of a PHA by using seawater in the past (see a list of media published on the website (https://www.nite.go.jp/nbrc/cultures/cultures/culture-list.html) by NBRC (National Institute of Technology and Evaluation, Biological Resource Center). However, the methods disclosed in such a report each only disclose a medium for the purpose of growing a highly halophilic bacterium, and do not relate to the production of the PHA. In addition, in the conventional techniques, concentrations of various components are kept at the initial or predetermined concentrations, and no attempt has been made to produce the PHA by using concentrated seawater. In other words, no method for producing the PHA by using seawater, in particular concentrated seawater, has been known so far.

Patent Document 1 (JP 2016-185087 A) teaches a method for diluting concentrated seawater with water and using it to culture algae. However, the document does not teach or suggest using dense seawater as it is or using a halophilic bacterium.

Patent Document 2 (JP 2018-133997 A) discloses a method for allowing a marine photosynthetic bacterium living in seawater areas to produce a PHA, use of seawater as a medium, and a less risk of contamination in a high-salinity medium. However, the document also teaches neither the halophilic bacterium nor dense seawater for use.

Patent Document 3 (JP 2014-512194 A) merely discloses a method for producing a salt containing useful components contained in algae, by using concentrated seawater.

Patent Document 4 (JP 2012-210165 A) teaches a PHA-producing bacterium isolated from deep sea; however, the composition used has only about the same concentration as that of seawater.

Non Patent Document 1 (Macromolecular Bioscience 2007, 7, 218) discloses that when Haloferax mediterranei is used and cultured, it is capable of producing 77.8 g/L of the PHA. However, the document does not describe seawater, and in addition, does not describe or suggest concentrated seawater (seawater that is concentrated).

Upon desalination of seawater, a problem of generation of a large amount of seawater that is denser than usual arises because fresh water is extracted from seawater. Currently, much of the dense seawater is discharged directly into the sea, and there are concerns that changes in the surrounding environment such as an increase in salinity, may have an adverse effect on the ecosystem.

Among the production methods of the present invention, the production method including concentrated seawater as seawater provides a biological production method for the PHA using dense seawater that is disposed of by a seawater desalination technique, or the like, thereby also achieving an effect of rendering the cost of preparing inorganic salts in a medium such as a salt reduced to a lower level than that by conventional methods.

Further, according to the present invention, an effect of a medium for culturing a halophilic bacterium (preferably a highly halophilic bacterium) being provided as a new utilization method for dense seawater that is currently disposed of as it is, is also achieved.

Characteristics of a water resource are that it is renewable but unevenly distributed in time and region, and in the regions suffering from water shortage, securing water for daily use is an unavoidable issue for a healthy and cultural life. In Japan as well, for example, there are many areas that have suffered from water shortage such as Inami Town in Hyogo Prefecture, Kagawa Prefecture, the Chita and Atsumi Peninsula in Aichi Prefecture, and the Kujukuri and Minami Boso regions in Chiba Prefecture, and water utilization equipment such as dams and canals for agricultural and domestic water, and utilization facilities such as a cylindrical diversion in order to distribute water accurately, have been built.

Today, in Japan, the problem of insufficient water for daily use has almost been solved, but there are regions overseas such as the Middle East, where water resources are limited and securing water for daily use by rainfall is difficult, for example. In such regions, agriculture is difficult in the first place, and agricultural products are procured through imports; however, methods for securing water for daily use such as seawater desalination, are powerful and important methods.

The present invention enables reuse of concentrated seawater, which is also a problem in the Middle East and other parts of the world, and contributes to protecting richness of the oceans in addition to enabling the production of useful materials at low cost.

Of the production methods of the present invention, according to the method using the highly halophilic bacterium (sometimes referred to as "Haloarchaea"), an effect of allowing for the production and recovery of the PHA at low cost is provided.

It has been known that there are some microorganisms producing a PHA among the types called highly halophilic bacteria that live only in a high-concentration sodium chloride aqueous solution. It has been known that bacterial cells that produce a PHA in the high-concentration sodium chloride aqueous solution are lysed in a low-concentration sodium chloride aqueous solution or in water after production, and the PHA stored in the cells can be taken out. Even though normally, when recovering the PHA from bacteria cells, a large amount of organic solvents and surfactants are used, in the case of the highly halophilic bacterium, only water is used to recover the PHA, which is further advantageous in terms of cost.

Being not bound by a theory, in the production method of the present invention, higher concentrations of salts than those of ordinary seawater or known media are presumed to promote growth of the target microorganism, thereby enabling the production efficiency of the PHA to be improved.

### Brief Description of Drawings

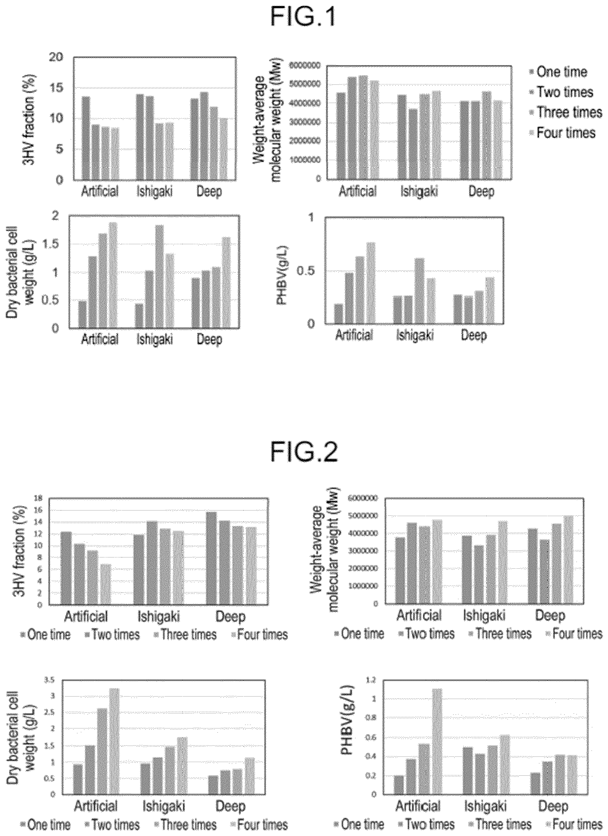

[Fig. 1] Fig. 1 is a graph of the 3HV fraction (%), the weight-average molecular weight (Mw), the dry bacterial cell weight (g/L), and the amount of PHBV produced (g/L) in Examples 1 to 12 (medium: the medium modified from #1214). In each graph, "Artificial" denotes each case of Examples 1 to 4 using artificial seawater, "Ishigaki" denotes each case of Examples 5 to 8 using natural seawater, and "Deep" denotes each case of Examples 9 to 12 using deep seawater, where the concentration factors of one time, two times, three times, and four times, respectively, as examples, are shown from left to right.
[Fig. 2] Fig. 2 is a graph of the 3HV fraction (%), the weight-average molecular weight (Mw), the dry bacterial cell weight (g/L), and the amount of PHBV produced (g/L) in Examples 13 to 24 (medium: the medium modified from #1338). In each graph, "Artificial" denotes each case of Examples 13 to 16 using artificial seawater, "Ishigaki" denotes each case of Examples 17 to 20 using natural seawater, and "Deep" denotes each case of Examples 21 to 24 using deep seawater, where the concentration factors of one time, two times, three times, and four times, respectively, as examples, are shown from left to right.

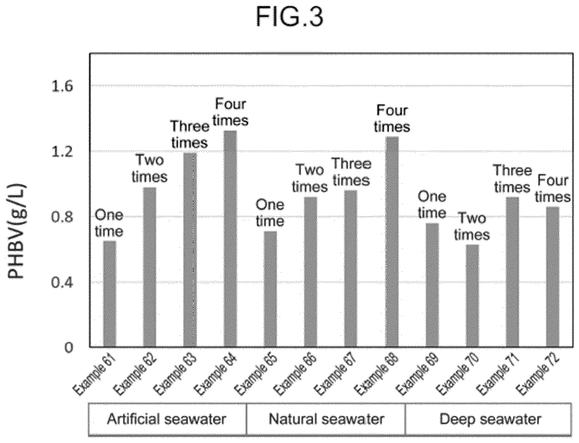

[Fig. 3] Fig. 3 is a graph of the amount of PHBV produced (g/L) for each of degrees of concentration (one time to four times) in Examples 61 to 64 (artificial seawater), 65 to 68 (natural seawater), and Examples 69 to 72 (deep seawater).

### Description of Embodiments

The present invention will be described in more detail below. The molecular weight of the PHA used herein is a value measured by gel permeation chromatography using a polystyrene standard, unless otherwise stated.

As used herein, "to" refers to a numerical range (including two numerical values connected by the "to") specified by a range between the two numerical values. For example, the description "200,000 to 6 million" refers to a range composed of all numerical values existing between 200,000 and 6 million, including 200,000 and 6 million. In order to convey the same meaning, the notation "200,000 or more and 6 million or less" may be used.

When a molecular weight of a polymer is numerically expressed in the present description, a numerical value specified by including a range of numerical values understood by a person skilled in the art, is intended.

The production method for the PHA of the present invention is as follows:
A production method for a polyhydroxyalkanoic acid comprising:
(a) culturing a microorganism by using a medium including seawater; and
(b) obtaining the polyhydroxyalkanoic acid as a culture product in the culture.

The seawater used in the present invention is not limited, and
(i) seawater obtained by using artificial seawater elements,
(ii) deep seawater, and
(iii) natural seawater
are exemplified.

The seawater used in the present invention may be compounded with one or more of the aforementioned seawaters (i) to (iii) in any compounding ratio.

An origin of (ii) deep seawater or (iii) natural seawater used in the present invention is not limited.

A type of (ii) deep seawater used in the present invention is not limited, and includes for example, seawater collected from a depth of about 200 m or deeper.

The (iii) natural seawater used in the present invention refers to seawater other than (ii) deep seawater among naturally derived seawater.

A content ratio of each metal salt in the naturally derived seawater is known to be generally constant regardless of the location of collection.

In the present invention, seawater that is concentrated as seawater (sometimes referred to as "concentrated seawater" or "dense seawater" as used herein) is preferably used.

In the present invention, seawater that is unconcentrated may also be used as an alternative for the concentrated seawater in the following. The production method of the present invention using the unconcentrated seawater does not require pretreatment of collected seawater and has advantages such as small workload and low cost.

The concentrated seawater in the present invention includes:
(1) a composition of seawater collected from sea in which only the water content is reduced, where the amount of the components contained remains almost unchanged;
(2) a composition obtained by concentrating seawater (composition) obtained from an artificial seawater composition, or a composition obtained by preparing the artificial seawater composition with a lower water content than a predetermined water content;
(3) a composition obtained by concentrating or diluting an aqueous solution containing a salt collected from places with salt water other than oceans such as a salt lake, to an appropriate or desired degree without changing the absolute amount of components contained, wherein the composition is adjusted to a salinity higher than that of ordinary seawater; and
(4) a composition having the composition similar to the compositions of (1) to (3) above, or a composition having the composition similar to the compositions of (1) to (3) above with an addition of components such as salts, and the like. The salinity in these compositions is higher than about 3.5 %.

In the present invention, the seawater collected from sea is not limited and includes seawater with a salinity of about 3.5%, as well as seawater with a salinity lower or higher than about 3.5%. The term "salinity" or "salt concentration" of the seawater or the like as used herein refers to a salt content of seawater or the like, containing approximately 78% of sodium chloride and approximately 21% to 22% of inorganic salts such as magnesium chloride, magnesium sulfate, calcium sulfate, and potassium chloride as other salt contents, which are normally used in the art, i.e., a concentration of the portion composed of the salts.

As the salt concentration of concentrated seawater, 3.5% to 20% is recited for example, and the concentration of 7% to 14% is preferable.

The percentage (%) used for indicating a concentration of seawater in the present invention is specified by w/v unless otherwise stated.

A composition per se, which has a relatively higher proportion of composition due to evaporation of water occurred in the process of using a medium prepared with seawater or a composition having a composition similar to the seawater in culturing a microorganism, is different from the composition as the concentrated seawater of the present invention.

In the present invention, when using a composition of the seawater collected from sea as concentrated seawater in which only the water content is reduced without changing the amount of the components contained, i.e., when using a composition obtained by concentrating the seawater, a method of concentrating is not limited, and may employ heat concentration or a method using a reverse osmosis membrane, or a concentrating method by an ion-exchange membrane electrodialysis method, or the like.

Water may be added as necessary to adjust the degree of concentration.

In the case of using artificial seawater in the present invention, the type of artificial seawater is not limited and may be prepared by using a commercially available salt mixture for artificial seawater. Examples of the commercially available salt mixture for artificial seawater include Daigo's Artificial Seawater SP for marine microalgae, Red Sea Salt, artificial seawater MARINE ART, artificial seawater TetraMarine Salt, and Instant Ocean.

As the concentrated seawater in the present invention, a composition obtained by concentrating deep seawater or natural seawater may be used.

When artificial seawater is used in the present invention, a medium containing a salt mixture for the artificial seawater and NaCl that is not derived from the salt mixture for the artificial seawater is preferred because it contributes to production of a high molecular weight PHA and an increase in an amount of PHA produced. In addition, the method of the present invention is also preferred, wherein a medium includes artificial seawater obtained by using a salt mixture for the artificial seawater at a concentration higher than that normally used for preparing artificial seawater.

When using the deep seawater or the natural seawater is used in the present invention, a medium containing NaCl that is not derived from the deep seawater or the natural seawater is preferred because it contributes to production of a high molecular weight PHA.

As an example of the medium in the case of using the artificial seawater in the present invention, media using known media, the compositions of which are partially defined by the components contained in the seawater are exemplified; a medium modified from No. 1214 or No. 1338 is preferred in a list of media published on the website (https://www.nite.go.jp/nbrc/cultures/cultures/culture-list.html) by NBRC (National Institute of Technology and Evaluation, Biological Resource Center). The compositions of these media are as follows:

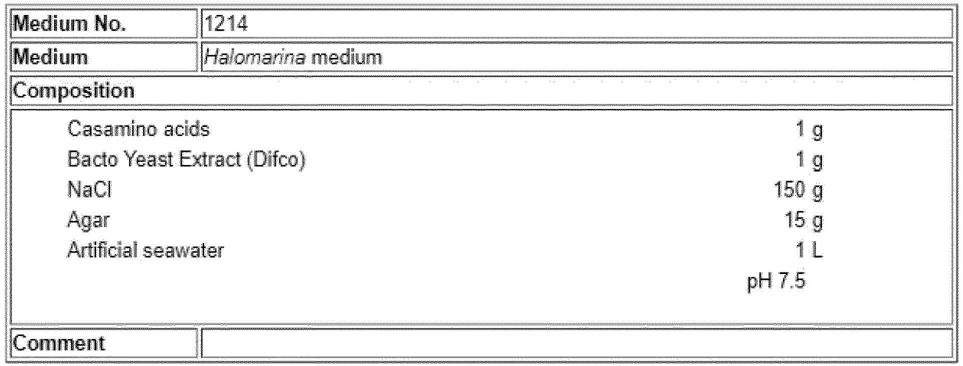

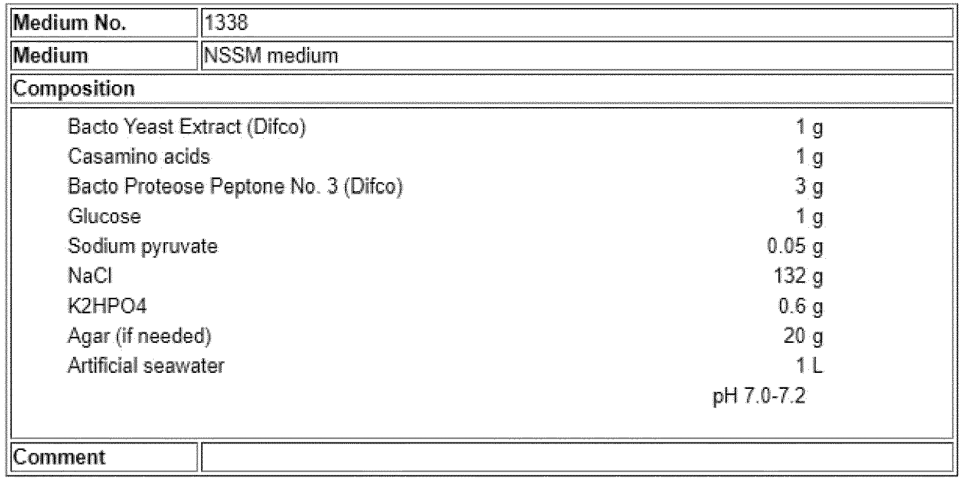

The medium containing the concentrated seawater in the present invention includes, in addition to the media using the compositions exemplified in (1) to (4) above, the media such that the amounts of trace metal salts contained in the media using the compositions exemplified in (1) to (4) above are about 1/32 to about 4 times the amount of known medium for microorganism culture.

An example of such a medium includes Daigo's Artificial Seawater SP for marine microalgae, which is artificial seawater with specified components. The concentrations (mg/L) of salts other than sodium chloride (trace metal salt and the like) in Daigo's Artificial Seawater SP are as follows.

| Metal salt | Concentration in medium (mg/1,000 mL) |
|---|---|
| MgCl₂·6H₂O | 9474 |
| CaCl₂·2H₂O | 1326 |
| Na₂SO₄ | 3505 |
| KCl | 597 |
| NaHCO₃ | 171 |
| KBr | 85 |
| Na₂B₄ O₇·10H₂O | 34 |
| SrCl₂ | 12 |
| NaF | 3 |
| LiCl | 1 |
| KI | 0.07 |
| CoCl₂·6H₂O | 0.0002 |
| AlCl₃·6H₂O | 0.008 |
| FeCl₃·6H₂O | 0.005 |
| Na₂WO₄·2H₂O | 0.0002 |
| (NH₄)₆ MO₇ O₂₄·4H₂O | 0.02 |
| MnCl₂·4H₂O | 0.0008 |

In the production method of the present invention, a medium containing one or more of these metal salts in an amount of 1.5 times or more each value in the above table may be used, and the medium preferably contains one or more thereof in an amount of 3 times or more, and more preferably in an amount of about 4 times or more. In addition, in the production method of the present invention, a medium containing one or more of the metals contained in these metal salts in the form of a salt or salts different from the metal salts indicated in the table, in an amount of 1.5 times or more the amount corresponding to that of the metal salts indicated by the values in the above table, may be used, and the medium preferably contains one or more of the metals in an amount of 3 times or more, and more preferably in an amount of about 4 times or more. By increasing the amount of the metal salts, the PHBV production efficiency can be enhanced and the molecular weight of the obtained PHBV can be increased.

In the production method of the present invention, the unconcentrated seawater or concentrated seawater may be used as it is, without adding one or more of the specific salts to seawater or a medium, or by reducing the amounts of the specific salts added. Such an embodiment allows the salts required as components of the medium to be replaced by the components of the seawater, which is advantageous and preferred in terms of cost and the labor required to prepare the medium. The salts that can be replaced are not limited, a calcium salt, a magnesium salt, a potassium salt, and iron chloride being exemplified.

In the production method of the present invention, to the unconcentrated or concentrated seawater can appropriately be added the salts to prepare a medium.

The salts to be added are not limited as long as they are necessary for growth of the microorganisms used, or they promote the growth thereof, and inorganic salts and organic salts are exemplified. Examples of such salts are exemplified as follow:
inorganic salts such as NH₄Cl, KH₂PO₄, K₂HPO₄, FeCl₃, NaCl, MgCl₂, MgSO₄, CaCl₂, KCl, NaHCO₃, NaBr and Na₂SO₄, and hydrates thereof;
organic salts such as sodium citrate, disodium citrate, monosodium glutamate, trisodium citrate and nitrilotriacetic acid, and hydrates thereof.

The types and the amounts of the salts described above in the medium used in the production method of the present invention are not limited. For example, for NH₄Cl, KH₂PO₄, FeCl₃, KCl, NaHCO₃, NaBr and NaCl among the inorganic salts, the following amounts (g/L) thereof are shown as examples:
NH₄Cl: 0.1 to 2.5, KH₂PO₄: 0.005 to 5.0, FeCl₃: 0.00001 to 0.01, KCl: 0.3 to 3, NaHCO₃: 0.01 to 0.3, NaBr: 0.03 to 0.5, and NaCl: 30 to 300.

A total salt concentration in the production method of the present invention includes, for example, 100 g/L to 300 g/L. The total salt concentration is preferably 150 g/L to 250 g/L.

As the medium used in the production method for PHA of the present invention, the media modified from the media of No. 1214, No. 1338, and No. 1380 are preferred in a list of media published on the website (https://www.nite.go.jp/nbrc/cultures/cultures/culture-list.html) by NBRC (National Institute of Technology and Evaluation, Biological Resource Center). The medium composition of No. 1380 is shown by quoting from the above medium list (the media compositions of No. 1214 and No. 1338 are as described above):

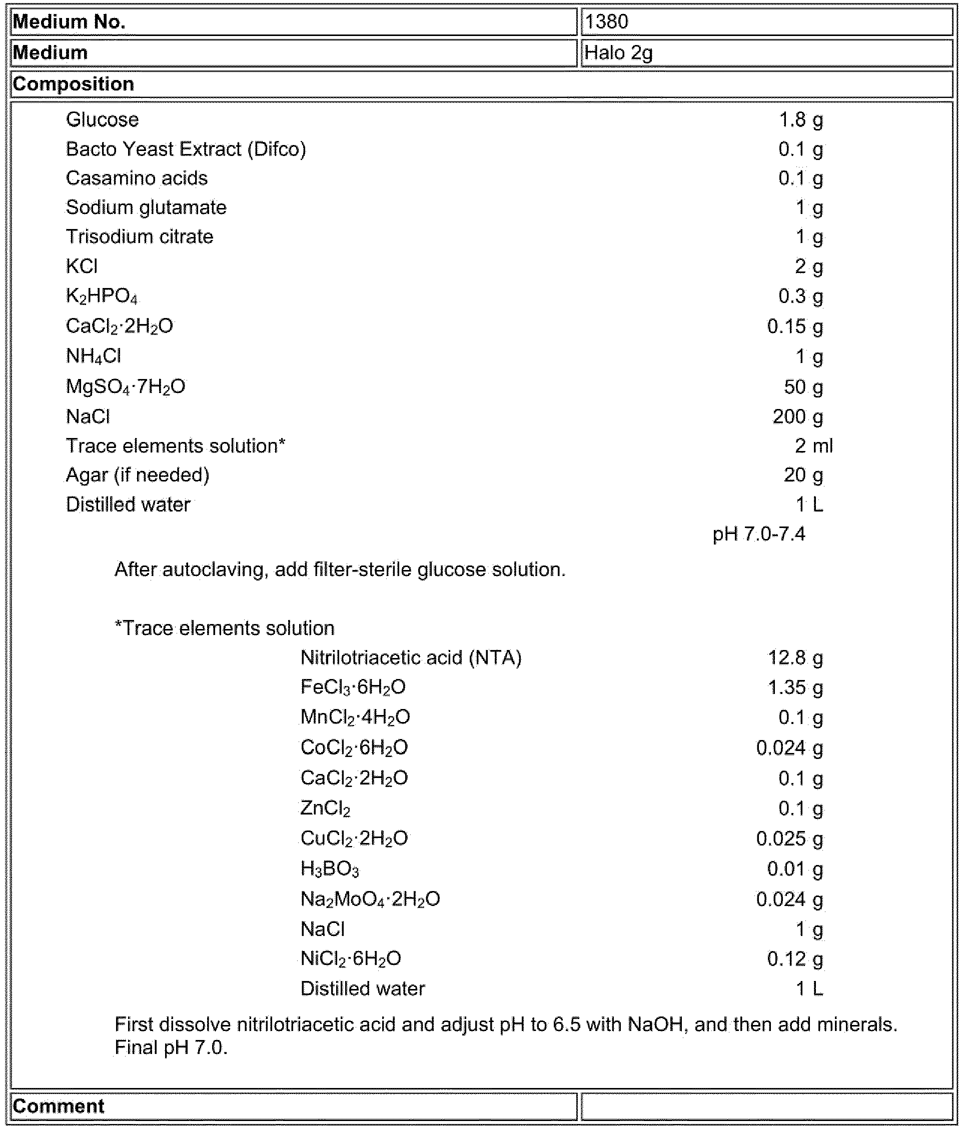

For example, when using seawater rich in eutrophication, it is preferably purified by a method of appropriately precipitating excessive nutrients such as phosphorus, or the like.

There is a possibility that other microorganisms and/or viruses may survive in the concentrated seawater. These other microorganisms and/or viruses are preferably removed before producing the PHA. The method of such removal is not limited and may be sterilization by adding an appropriate amount of the salts to increase the salinity, or by known methods such as autoclaving and filter sterilization.

In the production method of the present invention, sugar may be added to the medium. Said sugar does not include a sugar contained in other nutrient source such as a yeast extract. The sugars refer to those supplied separately from other nutrient source such as a yeast extract.

The sugars used in the production method of the present invention also include a derivative of each of the above sugars. As the derivative of sugar, an etherified or esterified sugar described above is assumed. In particular, the etherified derivative that is a methoxylated derivative, and the esterified derivative that is an acetylated derivative, are each exemplified.

In the production method of the present invention, a PHA can be produced by controlling a molecular weight of PHA to a weight-average molecular weight of 1 million to 6 million, for example, and by further adding a furfural compound as a carbon source, a PHA having a lower molecular weight can be obtained, and a PHA having a wider range of molecular weights can be produced. In the production method of the present invention further adding the furfural compound as a carbon source, a molecular weight of the PHA produced is not limited, and a PHA, for example, can be produced by controlling a molecular weight thereof to a weight-average molecular weight of 200,000 to 6 million. The production method of the present invention in which a furfural compound is used will further be described below.

As the production method of the present invention, the production method for a PHA by controlling a molecular weight of PHA to a weight-average molecular weight of 1 million to 5.75 million, is preferred.

### <Carbon source>

A carbon source may be added in the production method of the present invention.

As the carbon source, when using
(a) glucose and/or a derivative thereof alone,
   the molecular weight of the PHA produced tends to be large;
   as the carbon source, when using
(b) glucose and a derivative thereof as well as one or more of xylose, cellobiose, glucuronic acid, arabinose, mannose, galactose, sucrose, and derivatives thereof, the molecular weight of the PHA produced tends to be smaller than that in the case of (a) above; and as the carbon source, when using
(c) one or more of xylose, cellobiose, glucuronic acid, arabinose, mannose, galactose, sucrose, and a derivative thereof, and not using glucose and/or the derivative thereof, the molecular weight of the PHA produced tends to be further smaller than that in the case of (b) above.

An amount (initial concentration in the culture) of the sugar used in the production method of the present invention is not limited, and the amount of about 5 g/L to 910 g/L is exemplified. The amount of sugar is preferably about 5 g/L to about 500 g/L, and more preferably about 10 g/L to 50 g/L.

In the production method of the present invention, the sugar used may be fed in multiple times. The number of times and timing of feeding and a total amount fed are not limited; the number of times may be two, three, four, five, or six or more times. It is possible to feed the sugar continuously, in which case a method for controlling a concentration of sugar to be constant or controlling a feed rate to be constant, may be employed.

In the production method of the present invention, as described above, further adding a furfural compound as the carbon source enables the PHA to be produced by controlling a molecular weight of the PHA to a wider range of molecular weights, for example, a weight-average molecular weight of 200,000 to 6 million.

A furfural compound is a type of aldehyde produced from a carbohydrate as a raw material, and it is known that 5-hydroxymethylfurfural (HMF) is produced from hexose and furfural is produced from pentose, by heating the carbohydrates under an acidic condition or the like. Among furfural compounds, "furfural" is a common name for 2-furyl aldehyde.

The "furfural compound" in the present invention refers to furfural and 5-hydroxymethylfurfural as well as derivatives thereof.

Not being bound by any theory, the reason why a PHA can be produced by controlling a molecular weight of PHA over a wider range of molecular weights by the furfural compound, may be due to the furfural compound facilitating a transfer reaction of polymer chains formed by a polymerization; when a polymer is formed by polymerization, since elongation of the polymer chain is terminated by promoting transfer of a propagating end of a polymer chain having a relatively low molecular weight by the furfural compound and next elongation of a chain of a polymer different from said polymer is initiated, a molecular weight of the obtained PHA can possibly be smaller than that in the case where the furfural compound is not used.

The type of furfural compound used in the production method or control method of the present invention using the furfural compound, is not limited, and furfural, 5-hydroxymethylfurfural, and derivatives thereof are exemplified. Such derivatives include, for example, 5-hydroxymethylfurancarboxylic acid, 5-hydroxyfurfuryl alcohol, furan-2,5-bismethanol and an etherified or an esterified compound thereof. A methoxylated compound is exemplified as the etherified compound, and acetylated and methyl esterified compounds are each exemplified as the esterified compound.

The production method or the control method of the present invention is preferred if the methods use sugars that are glucose and a derivative thereof as well as one or more of xylose, cellobiose, glucuronic acid, arabinose, mannose, galactose, sucrose, and derivatives thereof, and furfural compounds that are furfural and/or 5-hydroxymethylfurfural or derivatives thereof.

An amount of the furfural compound used in the production method of the present invention is not limited, and an initial concentration thereof in a culture is exemplified by about 0.01 to about 3.0 g/L. The initial concentration of the furfural compound is preferably 0.01 g/L to 1 g/L.

A ratio of the amount of furfural compound used to an amount of a sugar used is not limited, and is exemplified by about 0.0025 to about 0.6. The production method or the control method of the present invention in which the ratio of the amount of the furfural compound used to the amount of the sugar used is 0.01 to 0.2, is preferred.

### <Microorganism>

The microorganism used in the production method of the present invention is not limited; a halophilic bacterium is suitable as the microorganism and a highly halophilic bacterium is particularly preferred. This is because the halophilic bacterium (including the highly halophilic bacterium) is a microorganism that is suitable for cultivation in the production method of the present invention and because the production capacity of the PHA is high and, furthermore, the PHA produced is easy to recover.

Among the highly halophilic bacteria, Haloferax genus, Halalkalicoccus genus, Haloarchaeobius genus, Haloarcula genus, Halobacterium genus, Halobaculum genus, Halococcus genus, Halogranum genus, Halomarina genus, Halorubrum genus, Haloterrigena genus, Natrialba genus, and Natronobacterium genus are preferred with Haloferax mediterranei being particularly preferred.

### <Other culture conditions>

In the culture of a microorganism in the production method for a PHA of the present invention, conditions other than the above carbon source and microorganism are not limited, and conditions usually used in the present technical field and known conditions may be used.

A medium used for the culture in the production method of the present invention may be a solid, liquid, or a gel, but a liquid medium is preferred from the viewpoint of production speed.

In the culture in the production method of the present invention, the total salt concentration includes for example, 100 g/L to 300 g/L. The total salt concentration of 150 g/L to 250 g/L is preferred as described above.

The medium to be used includes, for example, a medium containing the inorganic salts such as NH₄Cl, KH₂PO₄, FeCl₃, NaCl, MgCl₂, MgSO₄, CaCl₂, KCl, NaHCO₃, and NaBr. The types and the amounts of these inorganic salts are not limited, and for example, for NH₄Cl, KH₂PO₄, FeCl₃, KCl, NaHCO₃, NaBr, and NaCl, the following amounts (g/L) thereof are shown as examples, as described above:
NH₄Cl: 0.1 to 2.5, KH₂PO₄: 0.005 to 5.0, FeCl₃: 0.00001 to 0.01, KCl: 0.3 to 3, NaHCO₃: 0.01 to 0.3, NaBr: 0.03 to 0.5, and NaCl: 30 to 300.

As the medium used in the production method for a PHA of the present invention, a medium of No. 1380 is preferred in a list of media published on the website (https://www.nite.go.jp/nbrc/cultures/cultures/culture-list.html) by NBRC (National Institute of Technology and Evaluation, Biological Resource Center). The medium composition of No. 1380 is shown by quoting from the above medium list:

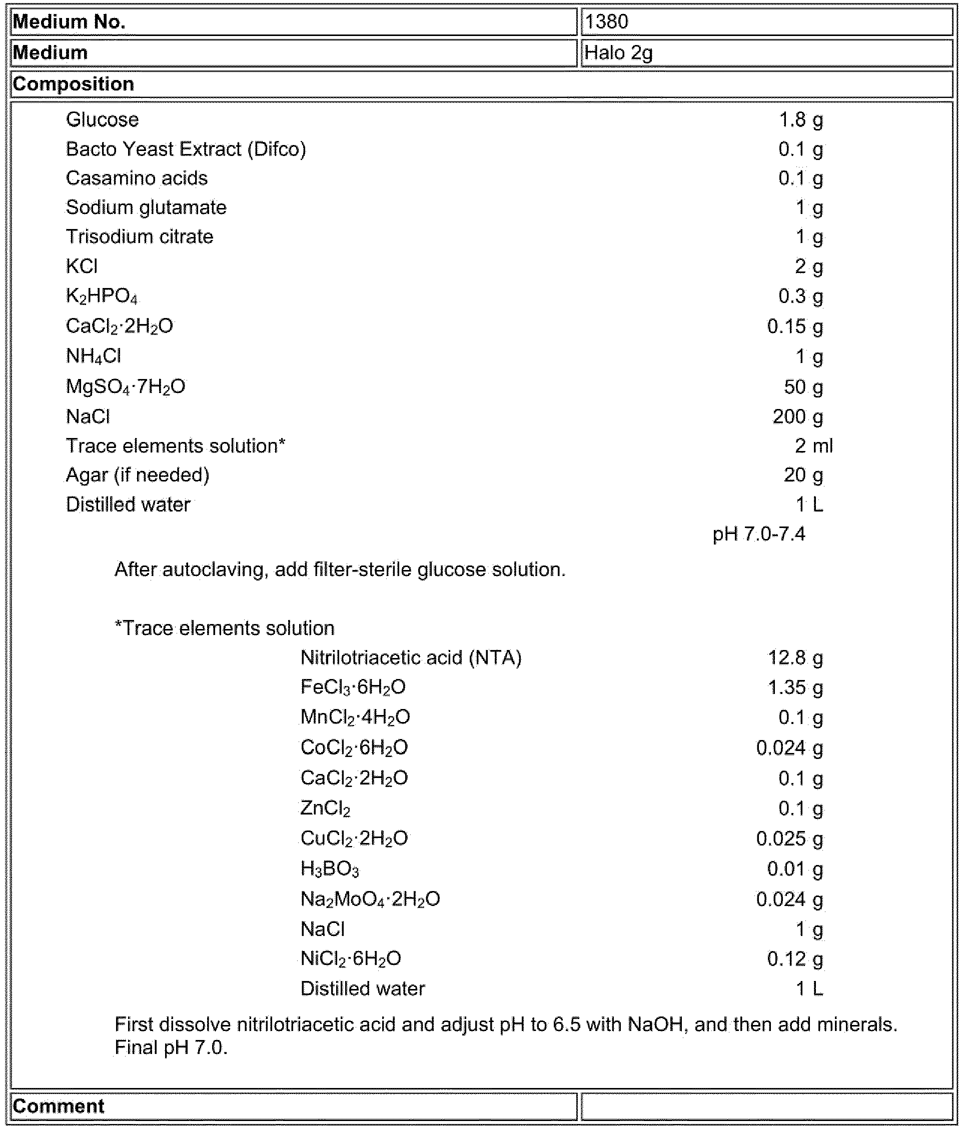

A nutrient source contained in a medium used for culturing a microorganism in the production method of the present invention will further be explained below; as described above, at least any one of the above sugars is used as the carbon source, and as a carbon source different from the aforementioned sugars for culturing a microorganism, carbon sources ordinarily used for culturing a microorganism such as amino acids, alcohols, fats and oils, and/or fatty acids and the like, may be used. Specifically, the following are exemplified:

As amino acids, glycine, alanine, phenylalanine, lysine, leucine, casamino acid, and glutamic acid;
as alcohols, methanol, ethanol, 1-propanol, 2-propanol, and glycerol;
as fats and oils, palm oil, palm kernel oil, corn oil, coconut oil, olive oil, soybean oil, and rapeseed oil; and
as fatty acids, acetic acid, propionic acid, butyric acid, valeric acid, caproic acid, crotonic acid, citric acid and pyruvic acid, as well as fatty acid salts such as a sodium salt and an ammonium salt thereof, are exemplified. When fatty acids are used, amounts of the fatty acids are preferably small.

The medium used for culturing a microorganism in the production method of the present invention may further comprise nitrogen sources, inorganic salts, and the like.

The nitrogen source includes, for example, ammonium salts such as ammonia, ammonium chloride, ammonium sulfate, and ammonium phosphate, as well as peptone, a meat extract, a yeast extract, and the like.

The inorganic salts include, for example, potassium dihydrogen phosphate, disodium hydrogen phosphate, magnesium phosphate, magnesium sulfate, sodium chloride, manganese(II) chloride, cobalt(II) chloride, calcium chloride, zinc chloride, copper(II) chloride, boric acid (H₃BO₃), sodium manganate (Na₂MnO₄), and nickel(II) chloride. In the present invention, various salts including these inorganic salts, may be used in the form of hydrates. Use of waste products such as scallop shells for a calcium salt, is preferred.

A temperature of culture when culturing microorganisms in the production method of the present invention is not limited provided that the microorganism can grow, and includes, for example, about 10°C to about 65°C, and it is preferably 20°C to 50°C , and more preferably 35°C to 45°C.

In the production method of the present invention, timings of adding carbon sources, inorganic salts, and organic nutrient sources are not limited; commonly used methods such as batch culture, fed-batch culture and continuous culture can appropriately be selected and used according to purposes.

A culture time for a microorganism in the production method of the present invention such as 24 hours to 168 hours for the batch culture, 72 hours to 14 days for the fed-batch culture, or a long period of longer than 14 days for the continuous culture, is exemplified without limitation.

In the production method of the present invention, a buffer solution can be added to render a pH difficult to change during culture. Said buffer solution includes a tris hydrochloric acid buffer solution, PIPES, and HEPES. The use of PIPES and HEPES is preferred because it is difficult for the buffer solution itself to be used as the nitrogen source, carbon source, or sulfide source upon culture.

The concentration of the buffer solution includes 5 g/L to 40 g/L, with 10 to 20 g/L being preferred.

When carrying out shaking culture for a medium containing a buffer solution by using a flask, the pH hardly changes without pH adjustment, and therefore, the conditions can be considered similar to those for a culture in a jar fermenter where a constant pH is always maintained.

In order to maintain a constant pH during the culture in the method of the present invention, basic components such as ammonia, potassium hydroxide, sodium hydroxide, and tripotassium phosphate may be added as needed.

In the production method of the present invention, it is preferred to increase an amount of dissolved oxygen in a medium, and it is more preferable to maintain the amount of dissolved oxygen in the medium at a saturated amount.

A pH of a medium is not limited in the production method of the present invention; an initial pH of the medium is preferably about 6.5 to about 7.5. It is more preferred that the initial pH of the medium is about 6.5 to about 7.5 and a pH of the medium being cultured is maintained at about 6.5 to about 7.5. The pH thereof is further preferably set to about 7.0 to about 7.4.

In the control method of the present invention also, other culture conditions used in the production method of the present invention can be employed.

### <PHA produced by production method of the present invention>

A type of PHA that is a product in the production method of the present invention is not limited; a PHBV, PHB (polyhydroxybutyrate), poly(hydroxybutyrate/hydroxyhexanoate, and poly(3-hydroxybutyrate/4-hydroxybutyrate) are exemplified. Of these, the PHBV and/or the PHB are suitable products.

The PHA produced by the production method of the present invention can be recovered from bacterial cells by known methods including centrifugation and filtration/precipitation using an organic solvent, a surfactant, a low-concentration sodium chloride aqueous solution or water.

An amount and a 3HV fraction of PHA produced by a microorganism in the production method of the present invention may be measured by using a known method such as a GC-MS method. When the PHA obtained by the production method of the present invention is a copolymer, the secondary component fraction may also be determined by using a known method such as the GC-MS method.

For measurement of concentrations of glucose or a derivative thereof, other carbohydrates, and furfural compounds, measurement using a commercially available concentration measurement kit or a known method such as an HPLC method may be applied.

When it is necessary to specify an amount of microbial cells produced in culturing the microorganism in the production method of the present invention, the amount of the microbial cells produced may be measured by known methods such as an absorbance method and a dry microbial cell weight measurement method.

A molecular weight of PHA obtained by the production method of the present invention may also be measured by a known method, for example, by gel permeation chromatography using a polystyrene standard. As described above, for the molecular weight of PHA in the present description, numerical values measured by gel permeation chromatography using a polystyrene standard are used unless otherwise specified.

The weight-average molecular weight of PHA produced by the production method of the present invention is about 1 million to about 6 million when the furfural compound is not used, and it is about 200,000 to about 6 million when the furfural compound is used. The molecular weight of PHA obtained in each range can be controlled or adjusted in the production method of the present invention.

### 2. PHBV

According to the production method of the present invention, a copolymer in which 3-hydroxybutanoic acid (3HB) and 3-hydroxyvaleric acid (3HV) are randomly copolymerized (hereinafter sometimes referred to as "PHBV"), is also provided and such a PHBV has a weight-average molecular weight of more than 3.61 million, and preferably more than 4 million. A PHBV of the present invention having a molecular weight of more than 5 million is more preferred, and a PHBV having a molecular weight of more than 5.7 million is further preferred.

The PHBV of the present invention comprises the structural units shown below as an arbitrary combination thereof in which a molecular weight of the entire PHBV exceeds 3.61 million wherein m and n represent integers in the formula below:

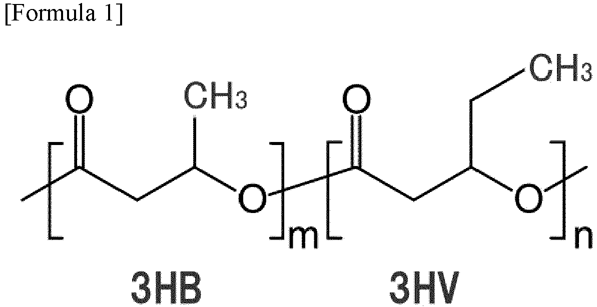

The structure, the composition of the monomer, and the molecular weight of PHBV of the present invention are not limited provided that the molecular weight exceeds 3.61 million. The PHBV of the present invention is linear, and the PHBV of the present invention, which is at least substantially linear, is preferred.

The PHBV of the present invention is a copolymer in which the 3HB and the 3HV are randomly copolymerized as described above, and a copolymer formed by alternating copolymerization or block copolymerization of the 3HB and the 3HV is also included.

In the PHBV of the present invention, a molar ratio of 3-hydroxybutanoic acid and 3-hydroxyvaleric acid constituting the PHBV is not limited. The PHBV of the present invention preferably has a 3HV fraction (a molar percentage of 3-hydroxyvaleric acid to the total moles of 3-hydroxybutanoic acid and 3-hydroxyvaleric acid constituting the copolymer) of about 5.0% to about 40.0%, the PHBV of the present invention more preferably has the 3HV fraction of about 5.0% to about 28.0%, the PHBV of the present invention still more preferably has the 3HV fraction of about 7.0% to 18.0%, and the PHBV of the present invention even still more preferably has the 3HV fraction of about 9.0% to about 15.0%.

Of the PHBVs of the present invention, a PHBV having characteristics superior to conventional PHBVs, in particular, a PHBV that is more flexible than the conventional PHBVs and/or is superior in strength, is preferable. Among the PHBVs of the present invention, a PHBV having an excellent tensile strength is preferable.

Of the PHBVs of the present invention, a PHBV having a tensile strength that is further improved by cold drawing, is also preferred. A PHBV having a tensile strength of 240 MPa or more after cold drawing is preferred, a PHBV having the tensile strength of 250 MPa or more is more preferred, and a PHBV having the tensile strength of 255 MPa or more is still more preferred.

According to the present invention, a cold-drawn film of a copolymer in which 3-hydroxybutanoic acid and 3-hydroxyvaleric acid are randomly copolymerized and having a weight-average molecular weight of more than 3.61 million, is also provided. Such a film is characterized in that it is superior in tensile strength as compared with a normal molecular weight PHBV having a weight-average molecular weight of 1 million.

Of the films of the present invention, a film having a tensile strength of 240 MPa or more is preferred, a film having the tensile strength of 250 MPa or more is more preferred, and a film having the tensile strength of 255 MPa or more is still more preferred.

Moreover, the aforementioned film obtained by cold drawing comprising the following steps:
(1) obtaining a molten film by melting a copolymer formed by random copolymerization of 3-hydroxybutanoic acid and 3-hydroxyvaleric acid;
(2) rapidly cooling the molten film obtained in (1) in ice water to make an amorphous film;
(3) drawing the amorphous film obtained in (2) as it is in ice water to a 10-fold length to orient the amorphous film; and
(4) subjecting the oriented film obtained in (3) to tension heat treatment to obtain a crystallized cold-drawn film
is preferred because a film having an excellent tensile strength can be more certainly obtained:
Considering improvement of tensile strength by cold drawing, a film having the 3HV fraction of 5% or more is preferred because the improvement of tensile strength by cold drawing is remarkable. This is presumably because crystals comprising a 3HV component at a fraction of 5% or more exhibit the same strength as a PHB and can be melted at 170°C, and mechanism by which the effects of the present invention are exerted, is not bound by such a theory. The PHBV of the present invention having the 3HV fraction of 5% to 15% is more preferred.

The method for producing the PHBV of the present invention is not limited as long as it is a method including the aforementioned steps (a) and (b), and may be a method capable of producing a PHBV having a weight-average molecular weight of more than 3.61 million.

As such a method, a method for culturing a microorganism to obtain the PHBV of the present invention as a culture product is exemplified, and a method using highly halophilic bacteria as microorganism in the aforementioned culture and using glucose and/or a derivative thereof as carbon source is preferred and the method using glucose is particularly preferred. This is because according to these methods, a PHBV having a weight-average molecular weight of more than 3.61 million can be efficiently produced, and the PHBV produced can easily be recovered.

In the case of producing the PHBV of the present invention by using the aforementioned production method for the PHA of the present invention, the saccharides as carbon source such as
(a) glucose and/or a derivative thereof alone, or
(b) glucose and the derivative thereof as well as one or more of xylose, cellobiose, glucuronic acid, arabinose, mannose, galactose, sucrose, and derivatives thereof,

### may be used.

The PHBV of the present invention is produced by using a yeast extract even without adding a carbon source other than carbon sources such as amino acids contained in the yeast extract. By using the carbon source other than the carbon sources such as amino acids contained in the yeast extract, the PHBV of the present invention can be produced at a lower cost.

### (Examples of present invention)

The present invention will be described in more detail by way of examples and reference examples. The present invention is not limited to such examples in any way.

In all the examples, experiments were carried out by using a strain Haloferax mediterranei NBRC 14739 provided by NBRC unless otherwise stated.

Unless otherwise stated in all the examples, after culturing the above bacteria under the methods and conditions shown in each example section, a molecular weight of PHA (PHBV) obtained by culture was measured by GPC (gel permeation chromatography) using a polystyrene standard and shown as a molecular weight.

An EcoSEC HLC-8320GPC manufactured by Tosoh Corporation was used for GPC measurement. The guard column used was a TSKgel guardcolumn Super HZ-H, and the columns used were two TSK gel Super HZM-H columns connected in series. Chloroform (0.6 mL/min) was used as a mobile phase, and a column temperature was 40°C. A sample concentration was about 0.5 mg/mL, and a sample injection volume was 10 µL. Polystyrene standards were used to prepare a calibration curve.

A 3-hydroxyvaleric acid (3HV) fraction of a copolymer and an amount of PHBV produced were measured by using gas chromatography-mass spectrometry (GC-MS method) (apparatus name: Agilent 6890 / 5973 GCMS System) as follows. More specifically, 2 ml of a sulfuric acid-methanol mixed solution (15:85) and 2 ml of chloroform were added to about 2 mg to 25 mg of dry bacterial cells, and the mixture was sealed and heated at 100°C for 140 minutes to obtain methyl ester of a polyester decomposition product. After adding 1 mL of water thereto with stirring, a chloroform layer was analyzed by the GC-MS method.

The method for measuring an amount produced, a molecular weight, and a 3HV fraction of these PHAs is usually used in the present technical field as a method for specifying the amount produced, the molecular weight, and the 3HV fraction of PHA. Moreover, the values of the amount produced, the molecular weight, and the 3HV fraction of PHA specified by these methods can be accurately compared with the values of the amount produced, the molecular weight, and the 3HV fraction of PHA described in known documents, respectively.

A dry bacterial cell weight can be measured by a known method such as a freeze-drying method. When it is desired to quantify inorganic salts contained in the dry bacterial cells, a method for thermally decomposing all organic substances other than the inorganic salts at an elevated temperature, may be used.

In the present examples, the artificial seawater, the natural seawater, and the deep seawater were used as the seawater. Daigo's Artificial Seawater SP was used for the artificial seawater, and the seawater from Ishigaki Island was used for the natural seawater. The composition of the artificial seawater was as described in Table 1. The components of the natural seawater and the deep seawater were as listed in Table 2; the components other than the compounds listed in Table 2 were not analyzed.

### [Composition of artificial seawater]

**[Table 1]**

| Metal salt | Concentration in medium (mg/1,000 mL) |
|---|---|
| MgCl₂·6H₂O | 9474 |
| CaCl₂·2H₂O | 1326 |
| Na₂SO₄ | 3505 |
| KCl | 597 |
| NaHCO₃ | 171 |
| KBr | 85 |
| Na₂B₄ O₇·10H₂O | 34 |
| SrCl₂ | 12 |
| NaF | 3 |
| LiCl | 1 |
| KI | 0.07 |
| CoCl₂·6H₂O | 0.0002 |
| AlCl₃·6H₂O | 0.008 |
| FeCl₃·6H₂O | 0.005 |
| Na₂WO₄·2H₂O | 0.0002 |
| (NH₄)₆Mο₇ O₂₄·4H₂O | 0.02 |
| MoCl₂·4H₂O | 0.0008 |
| NaCl | 20747 |

### [Components of natural water and deep seawater]

**[Table 2]**

| | Natural water (Ishigaki Island) | Deep seawater |
|---|---|---|
| Sodium chloride (g/L) | 23.79 | 23.45 |
| Sulfate ion (g/L) | 2.2 | 2.2 |
| Calcium (g/L) | 0.32 | 0.33 |
| Magnesium (g/L) | 1.1 | 1.1 |
| Potassium (g/L) | 0.32 | 0.32 |
| Bromide ion (mg/L) | 65 | 64 |
| Copper (mg/L) | <0.2^{a)} | <0.2 |

| | | |
|---|---|---|
| a) "<0.2" indicates less than 0.2 mg/L | | |

### Examples 1 to 12

### [Materials and methods]

For Haloferax mediterranei, each medium was prepared with reference to the NBRC-designated medium No. 1214 (the component composition of NBRC-designated medium No. 1214 is described above and below). The artificial seawater, the natural seawater, and the deep seawater were used as the seawaters for Examples 1 to 4, 5 to 8, and 9 to 12, respectively. In each of Examples 1 to 4, 5 to 8, and 9 to 12, the concentration factors of seawater were 1 time (ordinary seawater), 2 times, 3 times, and 4 times, respectively, and sodium chloride was further added in the amount shown in Table 3 to match the total salinity. 50 mL of medium was added to a volumetric triangular flask of 300 mL and cultured at 200 rpm for 72 h at 37°C.

### [Results]

The results obtained as shown in the following Tables (Tables 3 and 4, and Fig. 1), indicating that in any seawater, as each concentration of the seawater increased, the 3HV fraction decreased, the dry bacterial cell weight increased, the amount of PHBV produced increased, and the molecular weight was stable and high.

**[Table 3]**

| | Type of seawater | Concentration factor of seawater | Seawater (g/L) | Sodium chloride (g/L) | Casamino acid (g/L) | Yeast extract (g/L) | Glucose (g/L) |
|---|---|---|---|---|---|---|---|
| Example 1 | Artificial seawater | One time | 36 | 150 | 1 | 1 | 5 |
| Example2 | Artificial seawater | Two times | 72 | 114 | 1 | 1 | 5 |
| Examples | Artificial seawater | Three times | 108 | 78 | 1 | 1 | 5 |
| Example 4 | Artificial seawater | Four times | 144 | 42 | 1 | 1 | 5 |
| Example 5 | Natural seawater | One time | 36 | 150 | 1 | 1 | 5 |
| Examples | Natural seawater | Two times | 72 | 114 | 1 | 1 | 5 |
| Example 7 | Natural seawater | Three times | 108 | 78 | 1 | 1 | 5 |
| Examples | Natural seawater | One time | 144 | 42 | 1 | 1 | 5 |
| Example 9 | Deep seawater | One time | 36 | 150 | 1 | 1 | 5 |
| Example10 | Deep seawater | Two times | 72 | 114 | 1 | 1 | 5 |
| Example11 | Deep seawater | Three times | 108 | 78 | 1 | 1 | 5 |
| Example12 | Deep seawater | Four times | 144 | 42 | 1 | 1 | 5 |

**[Table 4]**

| | Type of seawater | Concentration | DCW (g/L) | PHA (g/L) | 3HV (mol%) | Mw (× 10⁶) | Mw/Mn |
|---|---|---|---|---|---|---|---|
| Example 1 | Artificial seawater | One time | 0.49 | 0.19 | 13.5 | 4.55 | 1.96 |
| Example 2 | Artificial seawater | Two times | 1.28 | 0.48 | 9.03 | 5.39 | 1.62 |
| Example 3 | Artificial seawater | Three times | 1.68 | 0.64 | 8.64 | 5.46 | 1.55 |
| Example 4 | Artificial seawater | Four time | 1.88 | 0.76 | 8.47 | 5.20 | 1.79 |
| ExampleS | Natural seawater | One time | 0.44 | 0.25 | 14.0 | 4.44 | 1.75 |
| Example 6 | Natural seawater | Two times | 1.03 | 0.26 | 13.6 | 3.70 | 1.92 |
| Example? | Natural seawater | Three times | 1.83 | 0.62 | 9.23 | 4.49 | 1.77 |
| Examples | Natural seawater | Four time | 1.32 | 0.43 | 9.33 | 4.65 | 1.76 |
| Example 9 | Deep seawater | One time | 0.90 | 0.28 | 13.2 | 4.11 | 1.71 |
| Example10 | seawater | Two times | 1.03 | 0.25 | 14.3 | 4.11 | 1.93 |
| Examplel 1 | Deep seawater | Three times | 1.09 | 0.31 | 11.9 | 4.62 | 1.73 |
| Example12 | seawater | Four time | 1.62 | 0.44 | 10.1 | 4.15 | 1.90 |

### Examples 13 to 24

### [Materials and methods]

For Haloferax mediterranei, each medium was prepared with reference to the NBRC-designated medium No. 1338 (the component composition of NBRC-designated medium No. 1338 is described above and below). The artificial seawater, the natural seawater, and the deep seawater were used as the seawaters for Examples 13 to 16, 17 to 20, and 21 to 24, respectively. In each of Examples 13 to 16, 17 to 20, and 21 to 24, the concentration factors of the seawater were 1 time (ordinary seawater), 2 times, 3 times, and 4 times, respectively, and sodium chloride was further added in the amount shown in Table 5 to match the total salinity. 50 mL of medium was added to a volumetric triangular flask of 300 mL and incubated at 200 rpm for 72 h at 37°C.

**[Table 5]**

| | Type of seawater | Concentration factor of seawater | Seawater (g/L) | Sodium chloride (g/L) | Casamino acid (g/L) | Yeast extract (g/L) | Peptone (g/L) | Sodium pyruvate (g/L) | K₂HPO₄(g/L) | Glucose (g/L) |
|---|---|---|---|---|---|---|---|---|---|---|
| Example! 3 | Artificial seawater | One time | 3δ | 132 | 1 | 1 | 3 | 1 | 1 | 5 |
| Example14 | Artificial seawater | Two times | 72 | 96 | 1 | 1 | 3 | 1 | 1 | 5 |
| Example15 | Artificial seawater | Three times | 108 | 60 | 1 | 1 | 3 | 1 | 1 | 5 |
| Example16 | Artificial seawater | Four times | 144 | 24 | 1 | 1 | 3 | 1 | 1 | 5 |
| Example17 | Natural seawater | One time | 36 | 132 | 1 | 1 | 3 | 1 | 1 | 5 |
| Example18 | Natural seawater | Two times | 72 | 96 | 1 | 1 | 3 | 1 | 1 | 5 |
| Example19 | Natural seawater | Three times | 108 | 60 | 1 | 1 | 3 | 1 | 1 | 5 |
| Example20 | Natural seawater | Four times | 144 | 24 | 1 | 1 | 3 | 1 | 1 | 5 |
| Example21 | seawater | One time | 36 | 132 | 1 | 1 | 3 | 1 | 1 | 5 |
| Example22 | seawater | Two times | 72 | 96 | 1 | 1 | 3 | 1 | 1 | 5 |
| Example23 | seawater | Three times | 108 | 60 | 1 | 1 | 3 | 1 | 1 | 5 |
| Example24 | Deep seawater | Four times | 144 | 24 | 1 | 1 | 3 | 1 | 1 | 5 |

### [Results]

The results obtained were as shown in the table below (Table 6 and Fig. 2), indicating that in any seawater, as each concentration of the seawater increased, the 3HV fraction decreased, the dry bacterial cell weight increased, the amount of PHBV produced increased, and the molecular weight was stable and high.

**[Table 6]**

| | Type of seawater | Concentration | (g/L) | PHA (g/L) | 3HV (mol%) | Mw (×10⁶) | Mw/Mn |
|---|---|---|---|---|---|---|---|
| Example 13 | Artificial seawater | One time | 0.92 | 0.20 | 12.3 | 3.78 | 2.61 |
| Example 14 | Artificial seawater | Two times | 1.50 | 0.37 | 10.3 | 4.60 | 1.73 |
| Example 15 | Artificial seawater | Three times | 2.61 | 0.53 | 9.17 | 4.40 | 1.95 |
| Example 16 | Artificial seawater | Four time | 3.24 | 1.11 | 6.81 | 4.77 | 2.14 |
| Example 17 | Natural seawater | One time | 0.92 | 0.50 | 11.85 | 3.88 | 1.84 |
| Example 18 | Natural seawater | Two times | 1.14 | 0.43 | 14.1 | 3.31 | 2.04 |
| Example 19 | Natural seawater | Three times | 1.46 | 0.52 | 12.9 | 3.91 | 2.04 |
| Example 20 | Natural seawater | Four time | 1.77 | 0.63 | 12.5 | 4.69 | 1.56 |
| Example 21 | Deep seawater | One time | 0.59 | 0.23 | 15.7 | 4.26 | 1.78 |
| Example 22 | Deep seawater | Two times | 0.75 | 0.35 | 14.2 | 3.63 | 1.93 |
| Example 23 | seawater | Three times | 0.79 | 0.42 | 13.3 | 4.55 | 1.62 |
| Example 24 | seawater | Four time | 1.13 | 0.41 | 13.2 | 5.03 | 1.42 |

### Comparative Example 1, Examples 25 to 36, Comparative Example 2, Examples 37 to 48 (replaceability of inorganic salts by seawater components)

### [Materials and methods]

For Haloferax mediterranei, a medium modified from the BSM medium (the BSM medium is described in Reference Example B.) (Examples 25 to 36) and a medium modified from the 1380 medium (Examples 37 to 48) were prepared as shown in the table below (Table 7).

In these Examples, among the inorganic salts contained in the BSM medium or 1380 medium, all the inorganic salts excluding the ammonium salt and phosphate salt (calcium salt, magnesium salt, potassium salt, and trace metal salts), were replaced with seawater. Comparative Examples 1 and 2 were prepared as comparative examples corresponding to the above Examples.

In more detail, seawater and sodium chloride as well as the ammonium salt and the phosphate salt, were combined for use as the salts, in each of Examples 25 to 48, without changing the types and amounts of organic nutrient sources. In Comparative Examples 1 and 2, the seawater was not used and only the same salts (sodium chloride, ammonium salt and phosphate salt) as in the Examples were added as the salts. 50 mL of medium was added to a volumetric triangular flask of 300 mL and cultured at 200 rpm for 72 h at 37°C.

**[Table 7]**

| | Type of seawater | Concentration factor of seawater | Seawater (g/L) | Sodium chloride (g/L) | Ammonium chloride (g/L) | Potassium dihydrogen phosphate (g/L) | Yeast extract (g/L) | Casamino acid (g/L) | Monosodium glutamate (g/L) | Sodium citrate dihydrate (g/L) | Glucose (g/L) |
|---|---|---|---|---|---|---|---|---|---|---|---|
| Comparative Example 1 | - | - | 0 | 239 | 2 | 0.0375 | 5 | | | | 5 |
| Comparative Example 2 | - | - | 0 | 239 | 2 | 0.0375 | 1 | 0.1 | 1 | 1 | 5 |
| Example 25 | Artificial seawater | One time | 36 | 203 | 2 | 0.0375 | 5 | | | | 5 |
| Example 26 | Artificial seawater | Two times | 72 | 167 | 2 | 0.0375 | 5 | | | | 5 |
| Examples 27 | Artificial seawater | Three times | 108 | 131 | 2 | 0.0375 | 5 | | | | 5 |
| Example 28 | Artificial seawater | Four times | 144 | 95 | 2 | 0.0375 | 5 | | | | 5 |
| Example 29 | Natural seawater | One time | 36 | 203 | 2 | 0.0375 | 5 | | | | 5 |
| Example 30 | Natural seawater | Two times | 72 | 167 | 2 | 0.0375 | 5 | | | | 5 |
| Example 31 | Natural seawater | Three times | 108 | 131 | 2 | 0.0375 | 5 | | | | 5 |
| Example 32 | Natural seawater | Four times | 144 | 95 | 2 | 0.0375 | 5 | | | | 5 |
| Example 33 | Deep seawater | One time | 36 | 203 | a | 0.0375 | 5 | | | | 5 |
| Example 34 | Deep seawater | Two times | 72 | 167 | 2 | 00375 | 5 | | | | 5 |
| Example 35 | Deep seawater | Three times | 108 | 131 | 2 | 0.0375 | 5 | | | | 5 |
| Example 36 | Deep seawater | Four times | 144 | 95 | 2 | 0.0375 | 5 | | | | 5 |
| Example 37 | Artificial seawater | One time | 36 | 203 | | 0.0375 | 1 | 0.1 | 1 | 1 | 5 |
| Example 38 | Artificial seawater | Two times | 72 | 167 | | 0.0375 | 1 | 0.1 | 1 | 1 | 5 |
| Example 39 | Artificial seawater | Three times | 108 | 131 | | 0.0375 | 1 | 0.1 | 1 | 1 | 5 |
| Example 40 | Artificial seawater | Four times | 144 | 95 | | 00375 | 1 | 0.1 | 1 | 1 | 5 |
| Example 41 | Natural seawater | One time | 36 | 203 | | 0.0375 | 1 | 0.1 | 1 | 1 | 5 |
| Example 42 | Natural seawater | Two times | 72 | 167 | | 0.0375 | 1 | 0.1 | 1 | 1 | 5 |
| Example 43 | Natural seawater | Three times | 108 | 131 | | 0.0375 | 1 | 0.1 | 1 | 1 | 5 |
| Example 44 | Natural seawater | Four times | 144 | 95 | | 0.0375 | 1 | 0.1 | 1 | 1 | 5 |
| Example 45 | Deep seawater | One time | 36 | 203 | | 00375 | 1 | 0.1 | 1 | 1 | 5 |
| Example46 | Deep seawater | Two times | 72 | 167 | | 0.0375 | 1 | 0.1 | 1 | 1 | 5 |
| Example 47 | Deep seawater | Three times | 108 | 131 | | 0.0375 | 1 | 0.1 | 1 | 1 | 5 |
| Example 48 | Deep seawater | Four times | 144 | 95 | | 0.0375 | 1 | 0.1 | 1 | 1 | 5 |

### [Results]

The results were as shown in the table able below (Table 8), indicating that the more PHAs with ultra-high molecular weight were produced by using the seawater, as compared to Comparative Examples 1 and 2. It is clarified that the inorganic salts other than sodium chloride could be replaced by seawater, indicating superiority in terms of cost and labor to prepare the medium.

On the other hand, the amount of PHBV produced, denoted as "PHA" in Table 8, was the same as those of Examples 1 to 24 (Table 4). This may be because the lowering of pH during the culture influenced the growth of the bacteria.

**[Table 8]**

| | Type of seawater | Concen-tration factor of seawater | DCW (g/L) | PHA (g/L) | 3HV (mol%) | Mw (× 10⁶) | Mw/Mn |
|---|---|---|---|---|---|---|---|
| Comparative Example 1 | - | - | 0.60 | 0.22 | 12.6 | 3.34 | 1.85 |
| Comparative Example 2 | - | - | 0.49 | 0.084 | 11.9 | 4.88 | 1.52 |
| Example 25 | Artificial seawater | One time | 2.28 | 1.11 | 7.40 | 4.25 | 1.74 |
| Example 26 | Artificial seawater | Two times | 2.69 | 0.94 | 7.37 | 3.94 | 2.04 |
| Example 27 | Artificial seawater | Three times | 3.71 | 1.76 | 6.07 | 4.48 | 1.69 |
| Example 28 | Artificial seawater | Four times | 4.09 | 2.10 | 6.15 | 4.30 | 1.73 |
| Example 29 | Natural seawater | One time | 3.90 | 1.77 | 7.05 | 4.09 | 1.76 |
| Example 30 | Natural seawater | Two times | 2.53 | 1.45 | 6.44 | 4.49 | 1.81 |
| Example 31 | Natural seawater | Three times | 2.50 | 0.92 | 7.36 | 3.28 | 1.99 |
| Example 32 | Natural seawater | Four times | 2.63 | 0.88 | 6.99 | 3.23 | 2.26 |
| Example 33 | Deep seawater | One time | 2.83 | 1.29 | 7.45 | 3.42 | 1.89 |
| Example 34 | Deep seawater | Two times | 2.06 | 0.85 | 8.20 | 3.22 | 2.10 |
| Example 35 | Deep seawater | Three times | 2.41 | 0.71 | 7.70 | 3.16 | 2.30 |
| Example 36 | Deep seawater | Four times | 2.29 | 0.70 | 7.41 | 3.18 | 2.00 |
| Example 37 | Artificial seawater | One time | 1.08 | 0.51 | 12.8 | 5.47 | 1.47 |
| Example 38 | Artificial seawater | Two times | 1.67 | 0.79 | 7.71 | 5.26 | 1.49 |
| Example 39 | Artificial seawater | Three times | 1.84 | 0.74 | 7.24 | 5.28 | 1.44 |
| Example 40 | Artificial seawater | Four times | 2.26 | 0.86 | 6.20 | 4.94 | 1.74 |
| Example 41 | Natural seawater | One time | 1.27 | 0.42 | 12.8 | 5.05 | 1.67 |
| Example 42 | Natural seawater | Two times | 1.27 | 0.46 | 11.5 | 5.37 | 1.41 |
| Example 43 | Natural seawater | Three times | 1.74 | 0.73 | 9.02 | 5.28 | 1.44 |
| Example 44 | Natural seawater | Four times | 1.95 | 1.01 | 7.39 | 5.22 | 1.46 |
| Example 45 | Deep seawater | One time | 0.99 | 0.42 | 13.2 | 4.65 | 1.92 |
| Example46 | Deep seawater | Two times | 1.26 | 0.47 | 12.4 | 5.30 | 1.54 |
| Example 47 | Deep seawater | Three times | 1.17 | 0.57 | 9.75 | 5.27 | 1.54 |
| Example 48 | Deep seawater | Four times | 1.63 | 0.58 | 8.38 | 5.63 | 1.46 |

### Comparative Examples 3 and 4, Examples 49 to 72 (effect of inhibition of change in pH)

### [Materials and methods]

Culture under conditions similar to those of a jar fermentor culture was carried out by using a medium without change in pH to confirm an effect achieved by inhibition of change in pH.

For Haloferax mediterranei, each of the media as shown in the following table (Table 9) (Comparative Example 3, Examples 49 to 60, Comparative Example 4, and Examples 61 to 72), was prepared. To obtain these media , to each medium corresponding to those of Comparative Example 1, Examples 25 to 36, Comparative Example 2, and Examples 37 to 48, was added with 15 g/L of PIPES as the buffer solution. 50 mL of the medium was added to a volumetric triangular flask of 300 mL and cultured at 200 rpm for 72 h at 37°C.

**[Table 9]**

| | Type of seawater | Concentration factor of seawater | Seawater (g/L) | Sodium chloride (g/L) | Ammonium chloride (g/L) | Potassium dihydrogen phosphate (g/L) | Yeast extract (g/L) | Casamino acid (g/L) | Monosodium glutamate (g/L) | Sodium citrate dihydrate (g/L) | Glucose (g/L) | PIPES (g/L) |
|---|---|---|---|---|---|---|---|---|---|---|---|---|
| Comparative Examples | - | - | 0 | 239 | 2 | 0.0375 | 5 | | | | 5 | 15 |
| Comparative Example 4 | - | - | 0 | 239 | 2 | 0.0375 | 1 | 0.1 | 1 | 1 | 5 | 15 |
| Example 49 | Artificial seawater | One time | 36 | 203 | 2 | 0.0375 | 5 | | | | 5 | 15 |
| Example 50 | Artificial seawater | Two times | 72 | 167 | 2 | 0.0375 | 5 | | | | 5 | 15 |
| Example 51 | Artificial seawater | Three times | 108 | 131 | 2 | 00375 | 5 | | | | 5 | 15 |
| Example 52 | Artificial seawater | Four times | 144 | 95 | 2 | 0.0375 | 5 | | | | 5 | 15 |
| Example 53 | Natural seawater | One time | 36 | 203 | 2 | 0.0375 | 5 | | | | 5 | 15 |
| Example 54 | Natural seawater | Two times | 72 | 167 | a | 0.0375 | 5 | | | | 5 | 15 |
| Example 55 | Natural seawater | Three times | 108 | 131 | 2 | 0.0375 | 5 | | | | 5 | 15 |
| Example 56 | Natural seawater | Four times | 144 | 95 | 2 | 0.0375 | 5 | | | | 5 | 15 |
| Example 57 | Deep seawater | One time | 36 | 203 | 2 | 0.0375 | 5 | | | | 5 | 15 |
| Example 58 | Deep seawater | Two times | 72 | 167 | 2 | 0.0375 | 5 | | | | 5 | 15 |
| Example 59 | Deep seawater | Three times | 108 | 131 | 2 | 0.0375 | 5 | | | | 5 | 15 |
| Example 60 | Deep seawater | Four times | 144 | 95 | 2 | 0.0375 | 5 | | | | 5 | 15 |
| Example 61 | Artificial seawater | One time | 36 | 203 | | 0.0375 | 1 | 0.1 | 1 | 1 | 5 | 15 |
| Example 62 | Artificial seawater | Two times | 72 | 167 | | 0.0375 | 1 | 0.1 | 1 | 1 | 5 | 15 |
| Example 63 | Artificial seawater | Three times | 108 | 131 | | 0.0375 | 1 | 0.1 | 1 | 1 | 5 | 15 |
| Example 64 | Artificial seawater | Four times | 144 | 95 | | 0.0375 | 1 | G.t | 1 | 1 | 5 | 15 |
| Example 65 | Natural seawater | One time | 36 | 203 | | 0.0375 | 1 | 01 | 1 | 1 | 5 | 15 |
| Example 66 | Natural seawater | Two times | 72 | 167 | | 0.0375 | 1 | 0.1 | 1 | 1 | 5 | 15 |
| Example 67 | 67 Natural seawater | Three times | 108 | 131 | | 0.0375 | 1 | 0.1 | 1 | 1 | 5 | 15 |
| Example 68 | Natural seawater | Four times | 144 | 95 | | 0.0375 | 1 | 0.1 | 1 | 1 | 5 | 15 |
| Example 69 | Deep seawater | One time | 36 | 203 | | 0.0375 | 1 | 0.1 | 1 | 1 | 5 | 15 |
| Example 70 | Deep seawater | Two times | 72 | 167 | | 0.0375 | 1 | 0.1 | 1 | 1 | 5 | 15 |
| Example 71 | Deep seawater | Three times | 108 | 131 | | 0.0375 | 1 | 0.1 | 1 | 1 | 5 | 15 |
| Example 72 | Deep seawater | Four times | 144 | 95 | | 0.0375 | 1 | 0.1 | 1 | 1 | 5 | 15 |

### [Results]

The results were as shown in the table below (Table 10-1) and Fig. 3. The changes in pH are shown together with the changes in pH in Comparative Example 1, Examples 25 to 36, Comparative Example 2, and Examples 37 to 48 (Table 10-2).

Compared to Comparative Examples 1 and 2, the more PHAs with ultra-high molecular weight were produced by using the seawater; in the case of both artificial seawater and natural seawater, the amount of PHA produced increased as the concentration of the seawater increased. Furthermore, the buffer solution inhibited the pH changes during the culture, which results in the higher amount of PHBV produced compared to those of Examples 25 to 48 (Table 10-1 and Fig. 3).

**[Table 10-1]**

| | Type of seawater | Concentration seawater factor of | DCW (g/L) | PHA (g/L) | 3HV (mol%) | Mw (× 10⁶) | Mw/Mn |
|---|---|---|---|---|---|---|---|
| Comparative Example 3 | - | - | 1.25 | 0.37 | 12.4 | 3.37 | 1.92 |
| Comparative Example 4 | - | - | 0.34 | 0.17 | 7.34 | 4.72 | 1.66 |
| Example 49 | Artificial seawater | One time | 2.92 | 1.16 | 6.69 | 4.26 | 1.74 |
| Example 50 | Artificial seawater | Two times | 3.86 | 1.97 | 6.53 | 4.57 | 1.72 |
| Example 51 | Artificial seawater | Three times | 3.87 | 1.89 | 6.38 | 4.50 | 1.70 |
| Example 52 | Artificial seawater | Four times | 3.79 | 2.02 | 6.39 | 4.69 | 1.71 |
| Example 53 | Natural seawater | One time | 3.30 | 1.52 | 7.65 | 3.90 | 1.75 |
| Example 54 | Natural seawater | Two times | 3.22 | 1.19 | 5.93 | 3.42 | 1.92 |
| Example 55 | Natural seawater | Three times | 3.82 | 1.81 | 6.82 | 3.64 | 1.83 |
| Example 56 | Natural seawater | Four times | 3.81 | 1.86 | 6.69 | 3.12 | 1.89 |
| Example 57 | Deep seawater | One time | 3.38 | 1.26 | 7.22 | 3.39 | 1.85 |
| Example 58 | Deep seawater | Two times | 3.13 | 1.01 | 5.94 | 3.27 | 1.86 |
| Example 59 | Deep seawater | Three times | 3.35 | 1.27 | 5.86 | 2.63 | 2.10 |
| Example 60 | Deep seawater | Four times | 2.59 | 0.74 | 7.60 | 2.93 | 2.14 |
| Example 61 | Artificial seawater | One time | 1.27 | 0.65 | 7.29 | 5.11 | 1.58 |
| Example 62 | Artificial seawater | Two times | 1.85 | 0.98 | 7.42 | 5.19 | 1.53 |
| Example 63 | Artificial seawater | Three times | 2.28 | 1.19 | 8.47 | 5.43 | 1.40 |
| Example 64 | Artificial seawater | Four times | 2.66 | 1.33 | 8.83 | 5.44 | 1.43 |
| Example 65 | Natural seawater | One time | 1.22 | 0.71 | 8.52 | 5.36 | 1.43 |
| Example 66 | Natural seawater | Two times | 1.67 | 0.92 | 8.83 | 5.06 | 1.67 |
| Example 67 | Natural seawater | Three times | 1.72 | 0.96 | 6.11 | 4.95 | 1.63 |
| Example 68 | Natural seawater | Four times | 2.51 | 1.29 | 7.73 | 5.02 | 1.67 |
| Example 69 | Deep seawater | One time | 1.44 | 0.76 | 8.06 | 5.14 | 1.66 |
| Example 70 | Deep seawater | Two times | 1.50 | 0.63 | 7.99 | 5.20 | 1.56 |
| Example 71 | Deep seawater | Three times | 1.74 | 0.92 | 7.77 | 5.28 | 1.44 |
| Example72 | Deep seawater | Four times | 1.75 | 0.86 | 7.29 | 5.07 | 1.70 |

**[Table 10-2]**

| | 0h | 48h | 72h |
|---|---|---|---|
| Comparative Example 1 | 7.2 | 6.27 | 6.2 |
| Comparative Example 2 | 7.2 | 6.48 | 6.75 |
| Example 25 | 7.2 | 6.23 | 6.03 |
| Example 26 | 7.2 | 6.75 | 6.58 |
| Example 27 | 7.2 | 6.94 | 6.56 |
| Example 28 | 7.2 | 7.09 | 6.86 |
| Example 29 | 7.2 | 6.33 | 6.05 |
| Example 30 | 7.2 | 6.45 | 5.71 |
| Example 31 | 7.2 | 6.53 | 6.13 |
| Example 32 | 7.2 | 6.65 | 6.47 |
| Example 33 | 7.2 | 6.26 | 6.15 |
| Example 34 | 7.2 | 6.24 | 6.01 |
| Example 35 | 7.2 | 6.39 | 6.1 |
| Example 36 | 7.2 | 6.48 | 6.04 |
| Example 37 | 7.2 | 6.78 | 6.41 |
| Example 38 | 7.2 | 5.46 | 5.26 |
| Example 39 | 7.2 | 6.52 | 5.39 |
| Example 40 | 7.2 | 6.48 | 7.49 |
| Example 41 | 7.2 | 6.49 | 6.87 |
| Example 42 | 7.2 | 6.6 | 6.77 |
| Example 43 | 7.2 | 5.34 | 5.1 |
| Example 44 | 7.2 | 5.21 | 5.01 |
| Example 45 | 7.2 | 6.29 | 5.78 |
| Example 46 | 7.2 | 5.39 | 5.18 |
| Example 47 | 7.2 | 5.31 | 5.08 |
| Example 48 | 7.2 | 5.23 | 5.06 |
| Comparative Example 3 | 7.2 | 6.92 | 6.83 |
| Comparative Example 4 | 7.2 | 6.99 | 6.99 |
| Example 49 | 7.2 | 6.85 | 6.77 |
| Example 50 | 7.2 | 6.77 | 6.78 |
| Example 51 | 7.2 | 6.82 | 6.89 |
| Example 52 | 7.2 | 6.89 | 7.09 |
| Example 53 | 7.2 | 6.75 | 6.79 |
| Example 54 | 7.2 | 6.66 | 6.47 |
| Example 55 | 7.2 | 6.78 | 6.68 |
| Example 56 | 7.2 | 6.83 | 6.74 |
| Example 57 | 7.2 | 6.79 | 6.64 |
| Example 58 | 7.2 | 6.69 | 6.4 |
| Example 59 | 7.2 | 6.58 | 6.88 |
| Example 60 | 7.2 | 6.9 | 6.59 |
| Example 61 | 7.2 | 6.98 | 6.98 |
| Example 62 | 7.2 | 7 | 6.97 |
| Example 63 | 7.2 | 7.02 | 6.96 |
| Example 64 | 7.2 | 7.01 | 6.98 |
| Example 65 | 7.2 | 6.96 | 6.95 |
| Example 66 | 7.2 | 6.94 | 6.89 |
| Example 67 | 7.2 | 6.99 | 6.94 |
| Example 68 | 7.2 | 7.14 | 7.12 |
| Example 69 | 7.2 | 6.99 | 7.02 |
| Example 70 | 7.2 | 6.83 | 6.8 |
| Example 71 | 7.2 | 6.94 | 6.88 |
| Example 72 | 7.2 | 6.99 | 6.95 |

### Preliminary culture test using concentrated seawater

For Haloferax mediterranei, the glucose concentration only was changed to 5 g/L in the NBRC-designated medium No. 1380 (the medium composition of the NBRC-designated medium No. 1380 is as described above). Further, the amount of the trace element solution fed in the NBRC-designated medium No. 1380 was changed as shown in the table below (Table 11). The culture was carried out for 72 hours in 50 mL of the total amount of the culture solution in a 300 mL Erlenmeyer flask.

**[Table 11]**

| | Glucose concentration (g/L) | Trace element solution (mL/1000mL) |
|---|---|---|
| Example 9-2 | 5 | 0.06 |
| Example 10-2 | 5 | 0.13 |
| Example 11-2 | 5 | 0.25 |
| Example 12-2 | 5 | 0.5 |
| Example 13-2 | 5 | 1 |
| Examplel4-2 | 5 | 2 (base) |
| Example 15-2 | 5 | 4 |
| Example 16-2 | 5 | 8 |

The composition of the trace element solution is as follows:

| | |
|---|---|
| Nitrilotriacetic acid (NTA) | 12.8 g |
| FeCl₃·6H₂O | 1.35 g |
| MnCl₂·4H₂O | 0.1 g |
| COCl₂·6H₂O | 0.024 g |
| CaCl₂·2H₂O | 0.1 g |
| ZnCl₂ | 0.1 g |
| CUCl₂·2H₂O | 0.025 g |
| H₃BO₃ | 0.01 g |
| Na₂MoO₄·2H₂O | 0.024 g |
| NaCl | 1 g |
| NiCl₂·6H₂O | 0.12 g |
| Distilled water | 1 L |

### Results

The results were as shown in the following table (Table 12).

**[Table 12]**

| | Trace element solution (mL/1000mL) | DGW(g/L) | PHA (g/L) | 3HV (mol%) | Mn (× 10⁶) | Mw (× 10⁶) | Mw/Mn |
|---|---|---|---|---|---|---|---|
| Example 9-2 | 0.06 | 1.09 | 0.39 | 11.3 | 3.30 | 5.31 | 1.61 |
| Example 10-2 | 0.13. | 1.94 | 0.74 | 10.5 | 3.77 | 5.65 | 1.50 |
| Example 11-2 | 0.25 | 2.84 | 1.23 | 10.8 | 3.56 | 5.53 | 1.55 |
| Example 12-2 | 0.5 | 2.93 | 1.03 | 18.2 | 3.88 | 5.60 | 1.45 |
| Example 13-2 | 1 | 2.83 | 0.88 | 24.9 | 4.03 | 5.80 | 1.44 |
| Example 14-2 | 2 (base) | 2.50 | 0.74 | 30.1 | 3.19 | 5.34 | 1.67 |
| Example 15-2 | 4 | 2.23 | 0.57 | 32.7 | 3.02 | 4.94 | 1.64 |
| Example 16-2 | 8 | 2.14 | 0.43 | 19.8 | 3.23 | 5.05 | 1.57 |

Each of Examples in which the concentrations of the salts included in the trace elements were relatively low (each of Examples 9-2 to 13-2) and relatively high (each of Examples 15-2 and 16-2) as compared to the concentration of NaCl, as assumed for the concentrated seawater used in the production method of the present invention, also produced the high molecular weight PHA: It was confirmed that, with the production method of the present invention, the high molecular weight PHA is produced.

In addition, by changing the amounts of trace elements (salts) contained in the concentrated seawater used in the production method of the present invention, the degree of growth of the microorganisms and the molecular weight of PHA produced are also clearly changed.

### Reference Example A

- (Materials and methods) For Haloferax mediterranei, culture was carried out with a total culture solution volume of 50 mL in a 300 mL Erlenmeyer flask using the NBRC-designated medium for the highly halophilic bacteria shown in Table 10 + 5 g/L of glucose. When glucose was contained in the designated medium, 5 g/L of glucose was further added in addition to the glucose originally contained.

The ingredients of each designated medium are shown below by quoting the medium list (https://www.nite.go.jp/nbrc/cultures/cultures/culture-list.html) published on the website of NBRC.

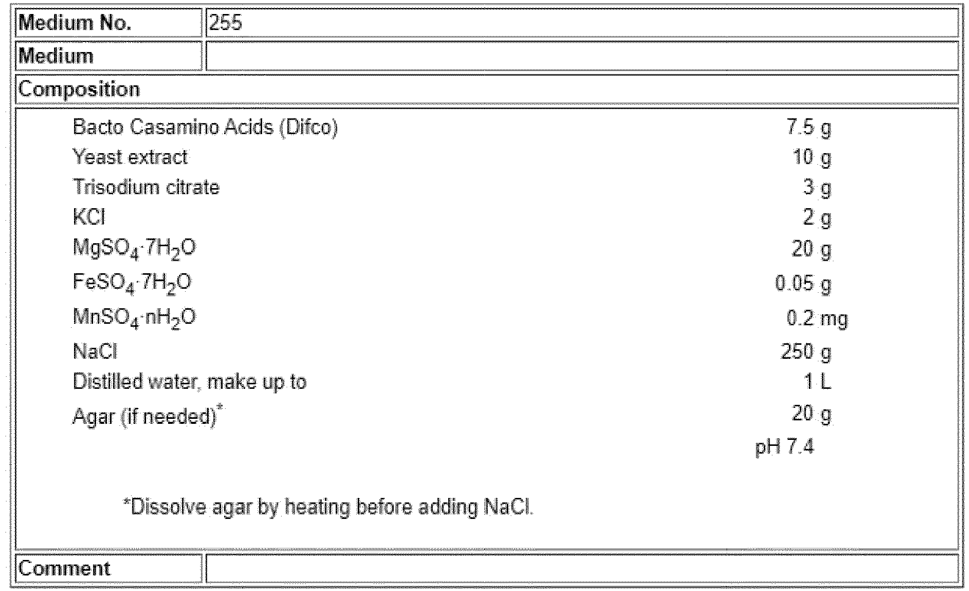

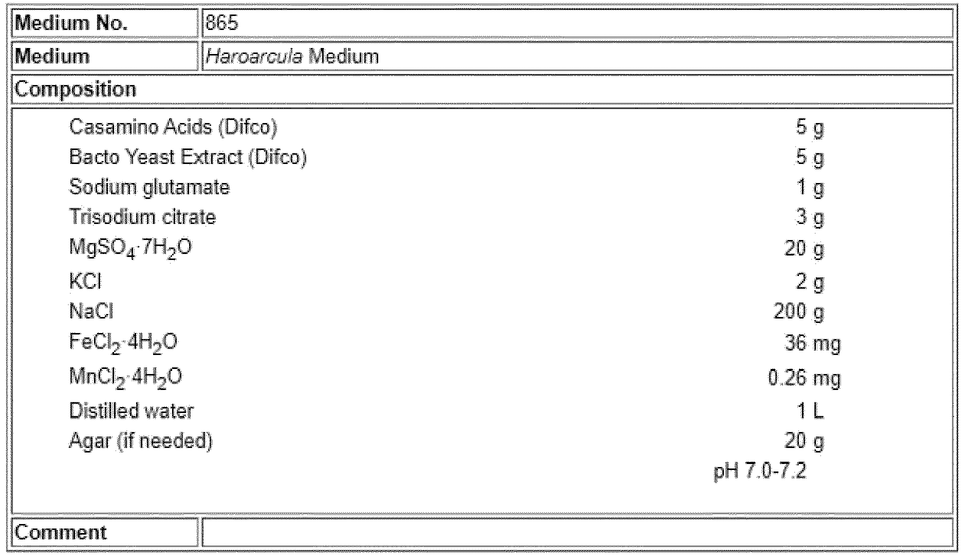

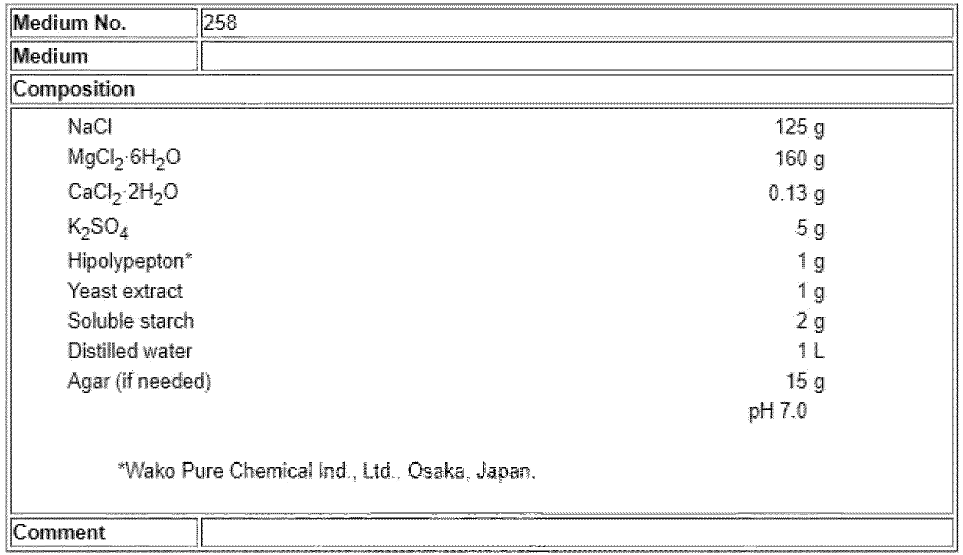

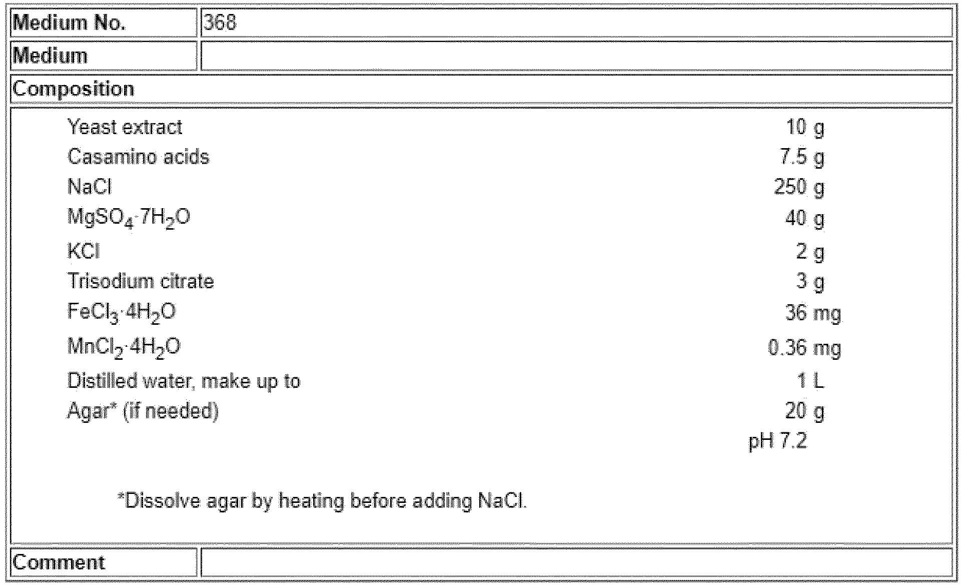

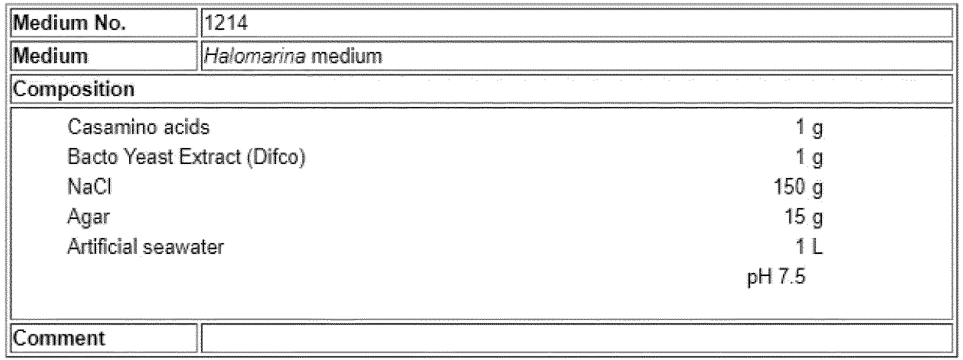

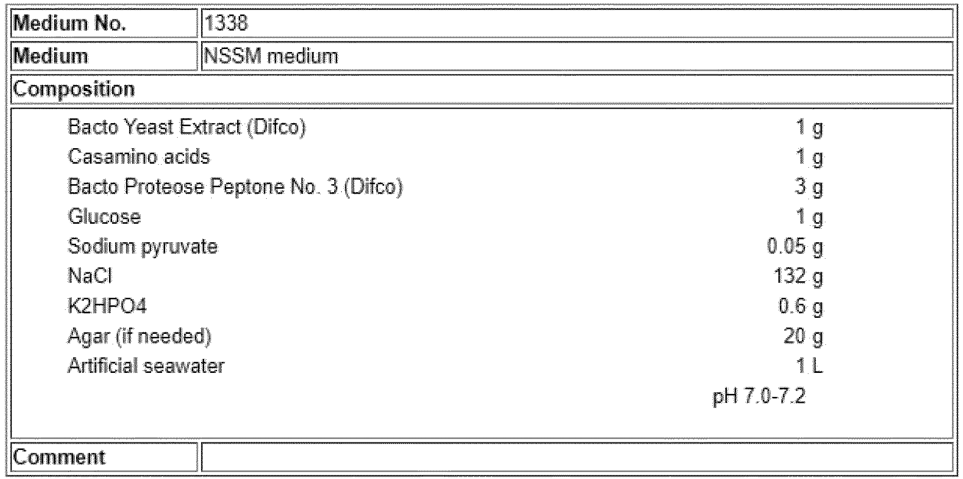

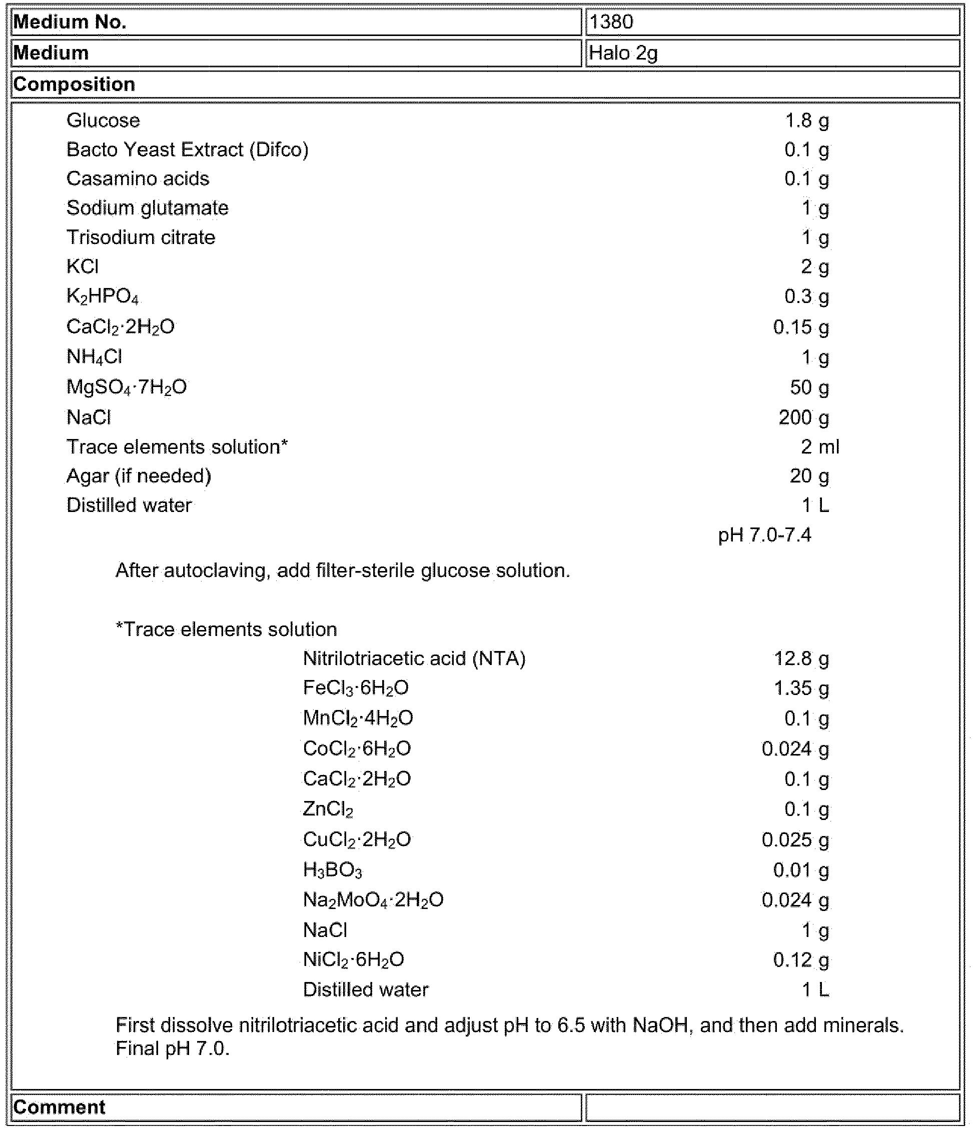

### • (Result)

The results were as shown in the table below (Table 13).

By changing the media, it was possible to produce the PHA with the weight-average molecular weight range of 1.3 million to 5.4 million.

In addition, the results clearly showed that the medium of NBRC No. 1380 could produce an unprecedented type of PHA having not only a high amount of PHA produced, but also a high 3HV fraction and a high molecular weight.

**[Table 13]**

| Culture | pH | DCW (g/L) | PHA (wt%) | 3HV (mol%) | *M*ₙ (×10⁶) | *M*_{w} (×10⁶) | *M*_{w}/*M*ₙ |
|---|---|---|---|---|---|---|---|
| BSM | 7.2 | 3.9 | 43.0 | 12. 1 | 2.7 | 4.4 | 1.6 |
| 255 | 7.2 | 5.7 | 27.5 | 16.6 | 0.94 | 1.6 | 1.7 |
| 865 | 7-7.2 | 2.8 | 33.7 | 27.4 | 1.5 | 2.6 | 1.8 |
| 258 | 7.0 | 3.7 | 34.8 | 14.5 | 2.4 | 4. 1 | 1. 7 |
| 368 | 7.2 | 4.3 | 3.8 | 27.2 | 0.81 | 1.3 | 1.6 |
| 1214 | 7.5 | 2.1 | 30.0 | 1:3.3 | 3.0 | 5.2 | 1.7 |
| 1338 | 7-7.2 | 1.2 | 24.1 | 17.1 | 2.3 | 4.5 | 1.9 |
| 1380 | 7-7.4 | 3.4 | 49.0 | 35.5 | 3.3 | 5.4 | 1.6 |

### Reference Example B

Flask culture of Haloferax mediterranei NBRC14739 was carried out at an initial glucose concentration of 10 g/L in the same manner as in Examples 1 to 12 except that a furfural compound was further used where a furfural concentration or an HMF (5-hydroxymethylfurfural) concentration was changed from 0.06 g/L to 1.6 g/L; the following shows more specific conditions.
- (Materials and methods) In a 300 mL Erlenmeyer flask, the following components were added so that a total volume of a culture solution was 50 mL, and shaking culture was carried out for 72 h at 37°C and 200 rpm. The initial pH was adjusted to 7.2.

Ingredients in the medium (g/L):
NH₄Cl 2, KH₂PO₄ 0.0375, FeCl₃ 0.005, NaCl 194,
MgCl₂ 16, MgSO₄ 24, CaCl₂ 1, KCl 5, NaHCO₃ 0.2,
NaBr 0.5, and a yeast extract 5.

To each medium (BSM medium) composed of the above components, predetermined amounts of the carbohydrate and the furfural compound shown in the table below were added as carbon sources and the culture was carried out for 72 h.
- (Results) The table below (Table 14) shows the 3HV fraction and the weight-average molecular weight, the polydispersity, the dry bacterial cell weight, and the amount of PHBV produced under each condition.

**[Table 14]**

| | Initial glucose concentration (g/L) | Initial furfural concentration (g/L) | Initial HMF concentration (g/L) | 3HV fraction (%) | Mw (× 10⁶) | Mw/Mn | Dry bacterial cell weight (g/L) | PHBV (g/L) |
|---|---|---|---|---|---|---|---|---|
| Example 1 | 10 | 0.06 | 0 | 13 | 1.2 | 2.15 | 4.07 | 2.15 |
| Example 2 | 10 | 0.1 | 0 | 12 | 0.46 | 1.95 | 5.56 | 1.95 |
| Example 3 | 10 | 0.2 | 0 | 10.5 | 0.26 | 2.27 | 4.26 | 2.27 |
| Example 4 | 10 | 0.4 | 0 | 17.2 | 0.31 | 2.06 | 1.88 | 2.06 |
| Example 5 | 10 | 1.6 | 0 | n.d | n.d | n.d | 0.274 | n.d |
| Example 6 | 10 | 0 | 0.06 | 13.5 | 3.38 | 1.68 | 3.76 | 1.68 |
| Example 7 | 10 | 0 | 0.1 | 14.6 | 0.69 | 1.8 | 4. 58 | 1.8 |
| Example 8 | 10 | 0 | 0.2 | 11.2 | 0.44 | 1.72 | 5.66 | 1.72 |
| Example 9 | 10 | 0 | 0.4 | 11.1 | 0.31 | 1.92 | 4.21 | 1.92 |
| Example 1 0 | 10 | 0 | 1.6 | n.d | n.d | n.d | 0.33 | n.d |

It is shown that by changing the concentration of furfural and the concentration of HMF, PHBVs having various molecular weights could be produced and the molecular weight could be controlled. The molecular weight of PHBV produced by the furfural compound such as furfural or HMF is to be controlled even when the sugars other than glucose is used. Moreover, it is considered that the furfural compound can be used in the production method for the polyhydroxyalkanoic acid of the present invention as well as in the control method of the molecular weight of the polyhydroxyalkanoic acid.

### Industrial Applicability

According to the present invention, the PHA can be produced while controlling the weight-average molecular weight between, for example, 200,000 to 6 million. Therefore, the present invention greatly contributes to the development of the PHA manufacturing industry and related industries.

## Claims

1. A production method for a polyhydroxyalkanoic acid, comprising:
(a) culturing a halophilic bacterium by using a medium including seawater; and
(b) obtaining the polyhydroxyalkanoic acid as a culture product in the culture.

2. The production method according to claim 1, comprising NaCl other than NaCl derived from seawater in a medium.

3. The production method according to claim 1 or 2, wherein the halophilic bacterium is a highly halophilic bacterium.

4. The production method according to any one of claims 1 to 3, wherein the highly halophilic bacterium is Haloferax mediterranei.

5. The production method according to any one of claims 1 to 4, wherein the polyhydroxyalkanoic acid is a copolymer formed by random copolymerization of 3-hydroxybutanoic acid and 3-hydroxyvaleric acid.

6. The production method according to claim 5, wherein a molar percentage of 3-hydroxyvaleric acid (3HV fraction) to the total moles of 3-hydroxybutanoic acid and 3-hydroxyvaleric acid constituting the copolymer is 5.0% to 40.0%.

7. The production method according to claim 6, wherein the polyhydroxyalkanoic acid is obtained with a sugar as a carbon source selected from:
(a) glucose and/or a derivative thereof alone,
(b) glucose and a derivative thereof as well as one or more of xylose, cellobiose, glucuronic acid, arabinose, mannose, galactose, sucrose and derivatives thereof, or
(c) one or more of xylose, cellobiose, glucuronic acid, arabinose, mannose, galactose, sucrose and a derivative thereof.

8. The production method according to any one of claims 1 to 7, wherein the polyhydroxyalkanoic acid has a weight-average molecular weight of more than 3.61 million.

9. The production method according to any one of claims 1 to 8, wherein the polyhydroxyalkanoic acid obtained has a weight-average molecular weight of 6 million or less.

10. The production method according to any one of claims 1 to 9, wherein the seawater comprises concentrated seawater.

11. The production method according to claim 10, wherein a salt concentration of the concentrated seawater is 3.5% to 20%.

12. The production method according to any one of claims 1 to 11, wherein the medium comprises at least one of the following component or seawater:
(i) artificial seawater elements,
(ii) deep seawater, and
(iii) natural seawater.

13. Use of seawater in a production method for a polyhydroxyalkanoic acid, said production method comprising:
(a) culturing a halophilic bacterium by using a medium comprising concentrated or unconcentrated seawater; and
(b) obtaining the polyhydroxyalkanoic acid as a culture product in the culture.

14. A production method for a polyhydroxyalkanoic acid, comprising culturing a halophilic bacterium by using a medium comprising seawater to obtain the polyhydroxyalkanoic acid as a culture product,
wherein a carbon source in the culture comprises a sugar and a furfural compound, the sugar being selected from:
(a) glucose and/or a derivative thereof alone,
(b) glucose and a derivative thereof as well as one or more of xylose, cellobiose, glucuronic acid, arabinose, mannose, galactose, sucrose, and derivatives thereof, or
(c) one or more of xylose, cellobiose, glucuronic acid, arabinose, mannose, galactose, sucrose, and a derivative thereof,
to obtain the polyhydroxy alkanoic acid.

15. A method for controlling a molecular weight of a polyhydroxyalkanoic acid obtained as a culture product by culturing a halophilic bacterium using a medium comprising seawater,
wherein a carbon source in the culture comprises a sugar and a furfural compound, the sugar being selected from:
(a) glucose and/or a derivative thereof alone,
(b) glucose and a derivative thereof as well as one or more of xylose, cellobiose, glucuronic acid, arabinose, mannose, galactose, sucrose, and derivatives thereof, or
(c) one or more of xylose, cellobiose, glucuronic acid, arabinose, mannose, galactose, sucrose, and a derivatives thereof,
thereby controlling the weight-average molecular weight of the polyhydroxyalkanoic acid produced.
